(19) Europäisches Patentamt
European Patent Office
Office européen des brevets

(11) EP 4 219 491 A1

(12) EUROPEAN PATENT APPLICATION
published in accordance with Art. 153(4) EPC

(43) Date of publication:
**02.08.2023 Bulletin 2023/31**

(21) Application number: **21872364.1**

(22) Date of filing: **17.09.2021**

(51) International Patent Classification (IPC):
***C07D 471/04*** (2006.01) ***A01M 1/20*** (2006.01)
***A01N 43/90*** (2006.01) ***A01N 47/02*** (2006.01)
***A01P 7/04*** (2006.01) ***A61K 31/437*** (2006.01)
***A61K 31/444*** (2006.01) ***A61P 33/00*** (2006.01)
***A61P 33/10*** (2006.01) ***A61P 33/14*** (2006.01)

(52) Cooperative Patent Classification (CPC):
**A01M 1/20; A01N 43/90; A01N 47/02; A01P 7/04; A61K 31/437; A61K 31/444; A61P 33/00; A61P 33/10; A61P 33/14; C07D 471/04**

(86) International application number:
**PCT/JP2021/034331**

(87) International publication number:
**WO 2022/065235 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.09.2020 JP 2020161237**
**28.05.2021 JP 2021090020**

(71) Applicant: **Nippon Soda Co., Ltd.**
**Tokyo 100-8165 (JP)**

(72) Inventors:
- **SAKIYAMA, Norifumi**
**Odawara-shi, Kanagawa 250-0280 (JP)**
- **KATO, Tetsuro**
**Odawara-shi, Kanagawa 250-0280 (JP)**
- **NISHIO, Fumiya**
**Odawara-shi, Kanagawa 250-0280 (JP)**
- **USHIJIMA, Daisuke**
**Odawara-shi, Kanagawa 250-0280 (JP)**
- **AOYAMA, Hikaru**
**Odawara-shi, Kanagawa 250-0280 (JP)**
- **KOBAYASHI, Tomomi**
**Odawara-shi, Kanagawa 250-0280 (JP)**
- **KAWAGUCHI, Masahiro**
**Odawara-shi, Kanagawa 250-0280 (JP)**
- **SAKANISHI, Keita**
**Odawara-shi, Kanagawa 250-0280 (JP)**

(74) Representative: **Sonnenhauser, Thomas Martin**
**Wuesthoff & Wuesthoff**
**Patentanwälte PartG mbB**
**Schweigerstraße 2**
**81541 München (DE)**

# (54) IMIDAZO[1,2-A]PYRIDINE COMPOUND AND PEST CONTROL AGENT

(57) The present invention provides an imidazo[1,2-a]pyridine compound that is excellent in pest control activity, particularly, insecticidal activity and/or acaracidal activity, is excellent in safety, and may be industrially advantageously synthesized. The imidazo[1,2-a]pyridine compound of the present invention is a compound of any of formulae (I) to (III) or a salt thereof. In the formulae, $R^1$ represents a substituted or unsubstituted C1-6 alkylsulfonyl group; $R^2$ and $R^3$ each independently represent a hydrogen atom, a substituted or unsubstituted C1-6 alkyl group or a halogeno group; R represents a substituted or unsubstituted C1-6 alkyl group, a substituted or unsubstituted C6-10 aryl group or the like; X represents a substituted or unsubstituted C1-6 alkyl group, a substituted or unsubstituted C2-6 alkenyl group, a substituted or unsubstituted C2-6 alkynyl group or the like; n represents a number of chemically acceptable Xs, and is an integer of 0 to 4; Xs are the same or different when n is 2 or more; and A represents a nitrogen atom or $CR^2$.

EP 4 219 491 A1

## Description

### Technical Field

The present invention relates to an imidazo[1,2-a]pyridine compound and a pest control agent. More specifically, the present invention relates to an imidazo[1,2-a]pyridine compound that has excellent insecticidal activity and/or acaracidal activity, is excellent in safety, and may be industrially advantageously synthesized, and a pest control agent containing the same as an active ingredient.

### Background Art

Various compounds having insecticidal or acaracidal activity have been proposed. For practical use of such compounds as agrochemicals, it is required not only to have sufficiently high efficacy but to be less likely to cause chemical resistance, to cause neither phytotoxicity to plants nor soil pollution, and to be low toxic to livestock, fishes, etc.

Patent document 1 discloses a compound of formula (A) etc.

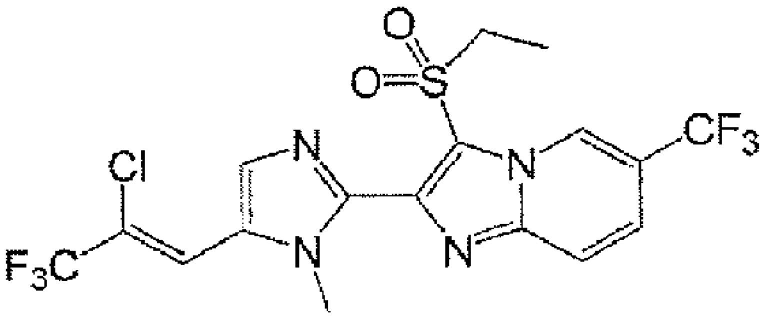

(A)

### Prior Art Documents

### Patent Documents

Patent document 1: WO2017/104741

### Summary of the Invention

### Object to be Solved by the Invention

An object of the present invention is to provide an imidazo[1,2-a]pyridine compound that is excellent in pest control activity, particularly, insecticidal activity and/or acaracidal activity, is excellent in safety, and may be industrially advantageously synthesized. Another object of the present invention is to provide a pest control agent, an insecticide or acaracide, an ectoparasite control agent, or an endoparasite control agent or expellant containing the imidazo[1,2-a]pyridine compound as an active ingredient.

### Means to Solve the Object

As a result of diligent studies to attain the objects, the present invention including the following form has been completed.

[1] A compound of any of formulae (I) to (III) or a salt thereof:

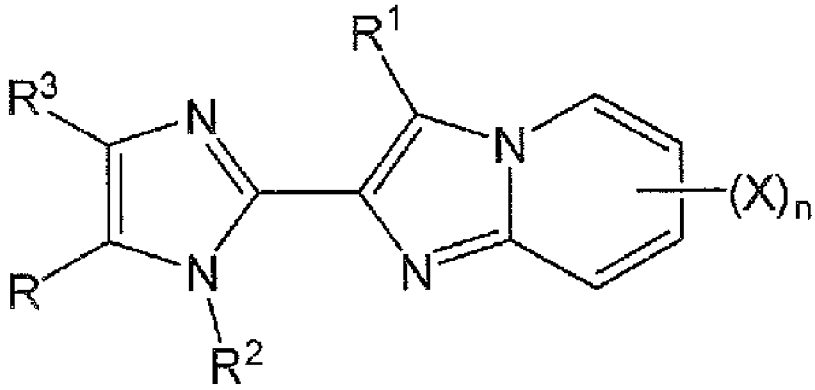

(I)

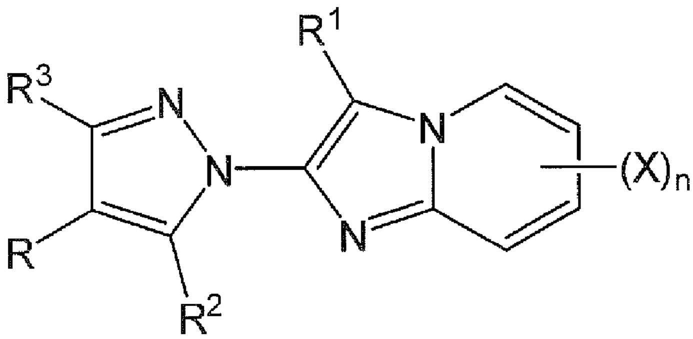

(II)

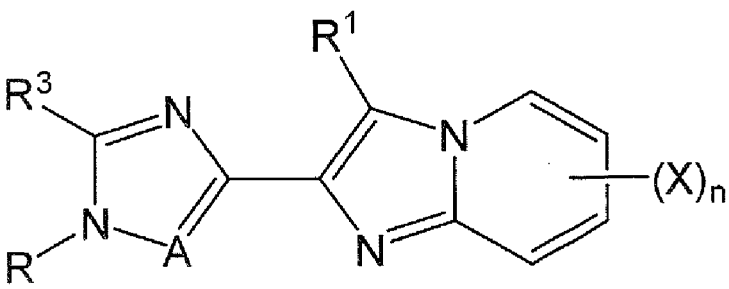

(III)

wherein

$R^1$ represents a substituted or unsubstituted C1-6 alkylsulfonyl group;

$R^2$ and $R^3$ each independently represent a hydrogen atom, a substituted or unsubstituted C1-6 alkyl group or a halogeno group;

R represents a substituted or unsubstituted C1-6 alkyl group, a substituted or unsubstituted C2-6 alkenyl group, a substituted or unsubstituted C6-10 aryl group or a substituted or unsubstituted 5- to 6-membered heteroaryl group;

X represents a substituted or unsubstituted C1-6 alkyl group, a substituted or unsubstituted C2-6 alkenyl group, a substituted or unsubstituted C2-6 alkynyl group, a hydroxyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 5- to 6-membered heteroaryl group, a substituted or unsubstituted C6-10 aryloxy group, a substituted or unsubstituted 5- to 6-membered heteroaryloxy group, a substituted or unsubstituted amino group, a substituted or unsubstituted aminocarbonyl group, a substituted or unsubstituted hydrazinyl group, a nitro group, a cyano group or a halogeno group;

n represents a number of chemically acceptable Xs, and is an integer of 0 to 4; Xs are the same or different when n is 2 or more; and

A represents a nitrogen atom or $CR^2$.

[2] A pest control agent comprising at least one active ingredient selected from the group consisting of a compound according to [1] and a salt thereof.

[3] An insecticide or acaracide comprising at least one active ingredient selected from the group consisting of a compound according to [1] and a salt thereof.

[4] An ectoparasite control agent comprising at least one active ingredient selected from the group consisting of a compound according to [1] and a salt thereof.

[5] An endoparasite control agent or expellant comprising at least one active ingredient selected from the group consisting of a compound according to [1] and a salt thereof.

## Effect of the Invention

The imidazo[1,2-a]pyridine compound of the present invention (a compound of any of formulae (I) to (III) or a salt thereof) may control pests that are problems associated with crops and hygiene. Particularly, the imidazo[1,2-a]pyridine compound of the present invention may effectively control agricultural insect pests and mites at a lower concentration. Furthermore, the imidazo[1,2-a]pyridine compound of the present invention may effectively control ectoparasites and endoparasites harmful to humans and animals.

## Mode of Carrying Out the Invention

[imidazo[1,2-a]pyridine compound]

The imidazo[1,2-a]pyridine compound of the present invention is a compound of formulae (I) to (III) (hereinafter, also referred to as compound (I) to (III)) or a salt thereof.

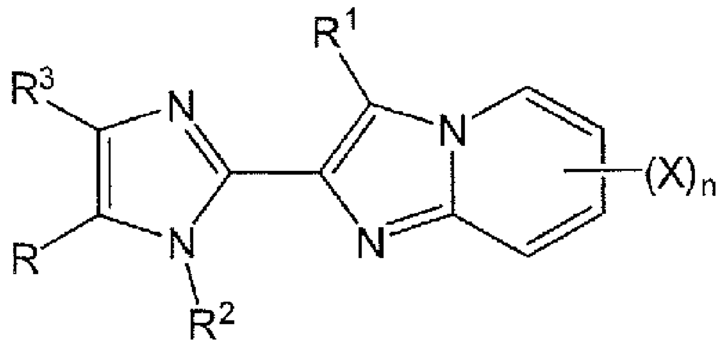

(I)

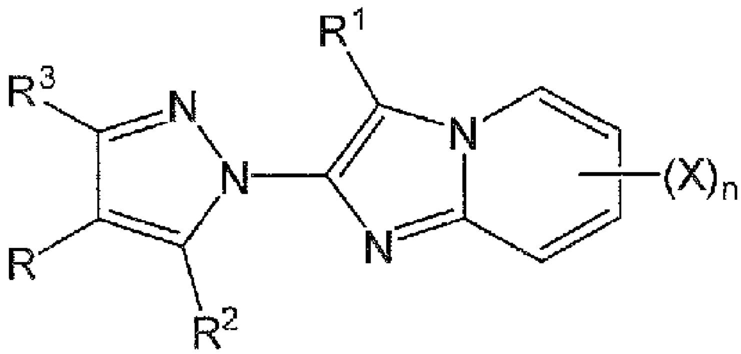

(II)

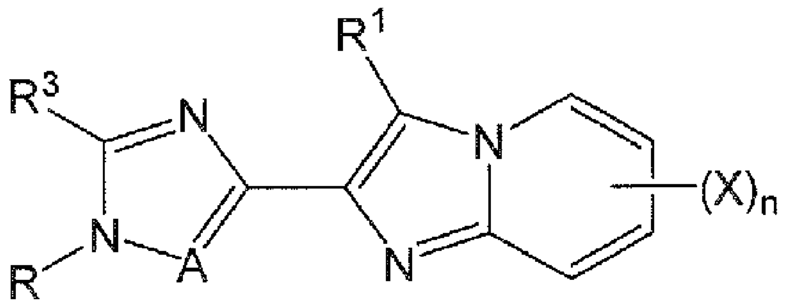

(III)

In the present invention, the term "unsubstituted" means a group consisting of only a mother nucleus. Only the name of a group consisting of a mother nucleus without the term "substituted" means an "unsubstituted" group unless otherwise specified.

On the other hand, the term "substituted" means that any hydrogen atom of a group consisting of a mother nucleus is substituted with a group (substituent) having a structure that is the same as or different from that of the mother nucleus. Thus, the "substituent" means another group bonded to the group consisting of a mother nucleus. The number of the substituent may be one or more. Two or more substituents are the same or different.

Terms such as "C1-6" mean that the number of carbon atoms in the group consisting of a mother nucleus is 1 to 6, etc. This number of carbon atoms does not include the number of carbon atoms present in the substituent. For example, a butyl group having an ethoxy group as a substituent is classified into a C2 alkoxy C4 alkyl group.

The "substituent" is not particularly limited as long as the substituent is chemically acceptable and produces the effect of the present invention. Hereinafter, a group capable of serving as the "substituent" is exemplified:

a C1-6 alkyl group such as a methyl group, an ethyl group, a n-propyl group, an i-propyl group, a n-butyl group, a s-butyl group, an i-butyl group, a t-butyl group, a n-pentyl group, and a n-hexyl group;
a C2-6 alkenyl group such as a vinyl group, a 1-propenyl group, a 2-propenyl group (allyl group), a 1-butenyl group, a 2-butenyl group, a 3-butenyl group, a 1-methyl-2-propenyl group, and a 2-methyl-2-propenyl group;
a C2-6 alkynyl group such as an ethynyl group, a 1-propynyl group, a 2-propynyl group, a 1-butynyl group, a 2-butynyl group, a 3-butynyl group, and a 1-methyl-2-propynyl group;
a C3-8 cycloalkyl group such as a cyclopropyl group, a cyclobutyl group, a cyclopentyl group, a cyclohexyl group, and a cubanyl group;
a C6-10 aryl group such as a phenyl group and a naphthyl group;
a C6-10 aryl C1-6 alkyl group such as a benzyl group and a phenethyl group;
a 3- to 6-membered heterocyclyl group;
a 3- to 6-membered heterocyclyl C1-6 alkyl group;
a hydroxy group;
a C1-6 alkoxy group such as a methoxy group, an ethoxy group, a n-propoxy group, an i-propoxy group, a n-butoxy group, a s-butoxy group, an i-butoxy group, and a t-butoxy group;
a C2-6 alkenyloxy group such as a vinyloxy group, an allyloxy group, a propenyloxy group, and a butenyloxy group;
a C2-6 alkynyloxy group such as an ethynyloxy group and a propargyloxy group;
a C6-10 aryloxy group such as a phenoxy group and a naphthoxy group;
a C6-10 aryl C1-6 alkoxy group such as a benzyloxy group and a phenethyloxy group;
a 5- or 6-membered heteroaryloxy group such as a thiazolyloxy group and a pyridyloxy group;
a 5- or 6-membered heteroaryl C1-6 alkyloxy group such as a thiazolylmethyloxy group and a pyridylmethyloxy group;
a formyl group;
a C1-6 alkylcarbonyl group such as an acetyl group and a propionyl group;
a formyloxy group;
a C1-6 alkylcarbonyloxy group such as an acetyloxy group and a propionyloxy group;
a C6-10 arylcarbonyl group such as a benzoyl group;
a C1-6 alkoxycarbonyl group such as a methoxycarbonyl group, an ethoxycarbonyl group, a n-propoxycarbonyl group, an i-propoxycarbonyl group, a n-butoxycarbonyl group, and a t-butoxycarbonyl group;
a C1-6 alkoxycarbonyloxy group such as a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy group;
a carboxyl group;
a halogeno group such as a fluoro group, a chloro group, a bromo group, and an iodo group;
a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a

perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, and a 2,4,6-trichlorohexyl group;

a C2-6 haloalkenyl group such as a 2-chloro-1-propenyl group and a 2-fluoro-1-butenyl group;

a C2-6 haloalkynyl group such as a 4,4-dichloro-1-butynyl group, a 4-fluoro-1-pentynyl group, and a 5-bromo-2-pentynyl group;

a C1-6 haloalkoxy group such as a trifluoromethoxy group, a 2-chloro-n-propoxy group, and a 2,3-dichlorobutoxy group;

a C2-6 haloalkenyloxy group such as a 2-chloropropenyloxy group and a 3-bromobutenyloxy group;

a C1-6 haloalkylcarbonyl group such as a chloroacetyl group, a trifluoroacetyl group, and a trichloroacetyl group;

an amino group;

a C1-6 alkyl-substituted amino group such as a methylamino group, a dimethylamino group, and a diethylamino group;

a C6-10 arylamino group such as an anilino group and a naphthylamino group;

a C6-10 aryl C1-6 alkylamino group such as a benzylamino group and a phenethylamino group;

a formylamino group;

a C1-6 alkylcarbonylamino group such as an acetylamino group, a propanoylamino group, a butyrylamino group, and an i-propylcarbonylamino group;

a C1-6 alkoxycarbonylamino group such as a methoxycarbonylamino group, an ethoxycarbonylamino group, a n-propoxycarbonylamino group, and an i-propoxycarbonylamino group;

an unsubstituted or substituted aminocarbonyl group such as an aminocarbonyl group, a dimethylaminocarbonyl group, a phenylaminocarbonyl group, and a N-phenyl-N-methylaminocarbonyl group;

an imino C1-6 alkyl group such as an iminomethyl group, a (1-imino)ethyl group, and a (1-imino)-n-propyl group;

a substituted or unsubstituted N-hydroxyimino C1-6 alkyl group such as a N-hydroxy-iminomethyl group, a (1-(N-hydroxy)-imino)ethyl group, a (1-(N-hydroxy)-imino)propyl group, a N-methoxy-iminomethyl group, and a (1-(N-methoxy)-imino)ethyl group;

a C1-6 alkoxyimino group such as a methoxyimino group, an ethoxyimino group, a n-propoxyimino group, an i-propoxyimino group, and a n-butoxyimino group;

an aminocarbonyloxy group;

a C1-6 alkyl-substituted aminocarbonyloxy group such as an ethylaminocarbonyloxy group and a dimethylaminocarbonyloxy group;

a mercapto group;

a C1-6 alkylthio group such as a methylthio group, an ethylthio group, a n-propylthio group, an i-propylthio group, a n-butylthio group, an i-butylthio group, a s-butylthio group, and a t-butylthio group;

a C1-6 haloalkylthio group such as a trifluoromethylthio group and a 2,2,2-trifluoroethylthio group;

a C6-10 arylthio group such as a phenylthio group and a naphthylthio group;

a 5- or 6-membered heteroarylthio group such as a thiazolylthio group and a pyridylthio group;

a C1-6 alkylsulfinyl group such as a methylsulfinyl group, an ethylsulfinyl group, and a t-butylsulfinyl group;

a C1-6 haloalkylsulfinyl group such as a trifluoromethylsulfinyl group and a 2,2,2-trifluoroethylsulfinyl group;

a C6-10 arylsulfinyl group such as a phenylsulfinyl group;

a 5- or 6-membered heteroarylsulfinyl group such as a thiazolylsulfinyl group and a pyridylsulfinyl group;

a C1-6 alkylsulfonyl group such as a methylsulfonyl group, an ethylsulfonyl group, and a t-butylsulfonyl group;

a C1-6 haloalkylsulfonyl group such as a trifluoromethylsulfonyl group and a 2,2,2-trifluoroethylsulfonyl group;

a C6-10 arylsulfonyl group such as a phenylsulfonyl group;

a 5- or 6-membered heteroarylsulfonyl group such as a thiazolylsulfonyl group and a pyridylsulfonyl group;

a C1-6 alkylsulfonyloxy group such as a methylsulfonyloxy group, an ethylsulfonyloxy group, and a t-butylsulfonyloxy group;

a C1-6 haloalkylsulfonyloxy group such as a trifluoromethylsulfonyloxy group and a 2,2,2-trifluoroethylsulfonyloxy group;

a tri-C1-6 alkyl-substituted silyl group such as a trimethylsilyl group, a triethylsilyl group, and a t-butyldimethylsilyl group;

a tri-C6-10 aryl-substituted silyl group such as a triphenylsilyl group;

a cyano group;

and a nitro group.

For these "substituents", any hydrogen atom in each substituent may be substituted with a group having a distinct structure. In this case, as the "substituent", a C1-6 alkyl group, a C1-6 haloalkyl group, a C1-6 alkoxy group, a

C1-6 haloalkoxy group, a halogeno group, a cyano group, a nitro group or the like may be exemplified.

The "3- to 6-membered heterocyclyl group" described above contains 1 to 4 heteroatoms selected from the group consisting of a nitrogen atom, an oxygen atom and a sulfur atom as ring-constituting atoms. The heterocyclyl group may be either monocyclic or polycyclic. The polycyclic heterocyclyl group has at least one hetero ring, and the remaining ring(s) may be any of a saturated alicyclic ring, an unsaturated alicyclic ring and an aromatic ring. As the "3- to 6-membered heterocyclyl group", a 3- to 6-membered saturated heterocyclyl group, a 5- or 6-membered heteroaryl group, a 5- or 6-membered partially unsaturated heterocyclyl group or the like may be exemplified.

As the 3- to 6-membered saturated heterocyclyl group, an aziridinyl group, an epoxy group, a pyrrolidinyl group, a tetrahydrofuranyl group, a thiazolidinyl group, a piperidyl group, a piperazinyl group, a morpholinyl group, a dioxolanyl group, a dioxanyl group or the like may be exemplified.

As the 5-membered heteroaryl group, a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group or the like may be exemplified.

As the 6-membered heteroaryl group, a pyridyl group, a pyrazinyl group, a pyrimidinyl group, a pyridazinyl group, a triazinyl group or the like may be exemplified.

[$R^1$]

In the formulae (I) to (III), $R^1$ represents a substituted or unsubstituted C1-6 alkylsulfonyl group.

As the "C1-6 alkylsulfonyl group" represented by $R^1$, a methylsulfonyl group, an ethylsulfonyl group, a t-butylsulfonyl group or the like may be exemplified.

As the substituents on the "C1-6 alkylsulfonyl group" represented by $R^1$, a halogeno group such as a fluoro group, a chloro group, a bromo group, and an iodo group may be preferably exemplified.

[$R^2$, $R^3$]

In the formulae (I) to (III), $R^2$ and $R^3$ each independently represent a hydrogen atom, a substituted or unsubstituted C1-6 alkyl group or a halogeno group.

The "C1-6 alkyl group" represented by $R^2$ and $R^3$ may be linear or branched. As the "C1-6 alkyl group", a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, an i-hexyl group or the like may be exemplified.

As the substituent on the "C1-6 alkyl group" represented by each of $R^2$ and $R^3$, halogeno groups such as a fluoro group, a chloro group, a bromo group and an iodo group may be preferably exemplified.

As the "halogeno group" represented by each of $R^2$ and $R^3$, a fluoro group, a chloro group, a bromo group, an iodo group or the like may be exemplified.

[R]

In the formulae (I) to (III), R is a substituted or unsubstituted C1-6 alkyl group, a substituted or unsubstituted C2-6 alkenyl group, a substituted or unsubstituted C6-10 aryl group or a substituted or unsubstituted 5- to 6-membered heteroaryl group.

As the "substituted or unsubstituted C1-6 alkyl group" represented by R, the same group as exemplified for $R^2$ and $R^3$ above is exemplified.

The "substituted C1-6 alkyl group" represented by R is preferably a C1-6 haloalkyl group.

As the "C1-6 haloalkyl group", a fluoromethyl group, a difluoromethyl group, a trifluoromethyl group, a 2,2,2-trifluoromethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, a 2,4,6-trichlorohexyl group or the like may be exemplified.

As the "C2-6 alkenyl group" represented by R, a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, a 5-hexenyl group or the like may be exemplified.

As the substituent on the "C2-6 alkenyl group", a halogeno groups such as a fluoro group, a chloro group, a

bromo group, and an iodo group; and C1-6 alkoxy groups such a methoxy group, an ethoxy group, a n-propoxy group, an i-propoxy group, a n-butoxy group, a s-butoxy group, an i-butoxy group and a t-butoxy group may be preferably exemplified.

The "C6-10 aryl group" represented by R means a monocyclic or polycyclic aromatic hydrocarbon group, and the polycyclic aryl group includes a partially saturated group in addition to a fully unsaturated group. For example, a phenyl group, a naphthyl group, an indenyl group, an indanyl group or the like may be exemplified.

The "5- to 6-membered heteroaryl group" represented by R means a 5- to 6-membered aromatic heterocyclic group having 1 to 4 nitrogen atoms, oxygen atoms or sulfur atoms as hetero atoms. For example, 5-membered heteroaryl groups such as a pyrrolyl group, a furyl group, a thienyl group, an imidazolyl group, a pyrazolyl group, an oxazolyl group, an isoxazolyl group, a thiazolyl group, an isothiazolyl group, a triazolyl group (specifically, a [1,2,3]triazolyl group or a [1,2,4]triazolyl group), an oxadiazolyl group (specifically, a [1,2,4]oxadiazolyl group or a [1,3,4]oxadiazolyl group), a thiadiazolyl group, and a tetrazolyl group; 6-membered heteroaryl groups such as a pyridyl group, a pyrazinyl group, a pyrimidinyl group, a pyridazinyl group and a triazinyl group; and the like may be exemplified.

As the substituents on the "C6-10 aryl group" and the "5- to 6-membered heteroaryl group" represented by R, halogeno groups such as a fluoro group, a chloro group, a bromo group and an iodo group; C1-6 alkyl groups such as a methyl group, an ethyl group, a n-propyl group, an i-propyl group, a n-butyl group, a s-butyl group, an i-butyl group, a t-butyl group, a n-pentyl group and a n-hexyl group; C1-6 haloalkyl groups such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group and a 2,4,6-trichlorohexyl group; C1-6 alkoxy groups such as a methoxy group, an ethoxy group, a n-propoxy group, an i-propoxy group, a n-butoxy group, a s-butoxy group, an i-butoxy group and a t-butoxy group; C1-6 haloalkoxy groups such as a 2-chloro-n-propoxy group, a 2,3-dichlorobutoxy group, and a trifluoromethoxy group may be exemplified.

[X]

In the formulae (I) to (III), X is a substituted or unsubstituted C1-6 alkyl group, a substituted or unsubstituted C2-6 alkenyl group, a substituted or unsubstituted C2-6 alkynyl group, a hydroxyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 5- to 6-membered heteroaryl group, a substituted or unsubstituted C6-10 aryloxy group, a substituted or unsubstituted 5- to 6-membered heteroaryloxy group, a substituted or unsubstituted amino group, a substituted or unsubstituted aminocarbonyl group, a substituted or unsubstituted hydrazinyl group, a nitro group, a cyano group or a halogeno group.

As the "substituted or unsubstituted C1-6 alkyl group" and the "halogeno group" represented by X, the same groups as exemplified for $R^2$ and $R^3$ above are exemplified.

As the "C1-6 alkylsulfonyl group" represented by X, the same group as exemplified for $R^1$ above is exemplified.

As the "substituted or unsubstituted C2-6 alkenyl group", the "substituted or unsubstituted C6-10 aryl group" and the "substituted or unsubstituted 5- to 6-membered heteroaryl group" represented by X, the same groups as exemplified for R above are exemplified.

As the "C2-6 alkynyl group" represented by X, an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, a 1,1-dimethyl-2-butynyl group or the like may be exemplified.

As the "C1-6 alkoxy group" represented by X, a methoxy group, an ethoxy group, a n-propoxy group, an i-propoxy group, a n-butoxy group, a s-butoxy group, an i-butoxy group, a t-butoxy group or the like may be exemplified.

As the "C1-6 alkoxycarbonyl group" represented by X, a methoxycarbonyl group, an ethoxycarbonyl group, a n-propoxycarbonyl group, an i-propoxycarbonyl group, a n-butoxycarbonyl group, a t-butoxycarbonyl group or the like may be exemplified.

As the "C1-6 alkylthio group" represented by X, a methylthio group, an ethyl thio group, a n-propylthio group, a n-butylthio group, a n-pentylthio group, a n-hexylthio group, an i-propylthio group, an i-butylthio group or the like may be exemplified.

As the "C1-6 alkylsulfinyl group" represented by X, a methylsulfinyl group, an ethylsulfinyl group, a t-butylsulfinyl group or the like may be exemplified.

As the substituent on the "C1-6 alkyl group" represented by X, a halogeno group such as a fluoro group, a chloro group, a bromo group or an iodo group; a C1-6 alkoxy group such as a methoxy group, an ethoxy group, a n-propoxy group, an i-propoxy group, a n-butoxy group, a s-butoxy group, an i-butoxy group or a t-butoxy group; or a C1-6 alkoxyimino group such as a methoxyimino group, an ethoxyimino group, a n-propoxyimino group, an i-propoxyimino group or a n-butoxyimino group may be preferably exemplified.

As the substituents on the "C2-6 alkenyl group", the "C2-6 alkynyl group", the "C1-6 alkoxy group", the "C1-6 alkylthio group", the "C1-6 alkylsulfinyl group" and the "C1-6 alkylsulfonyl group" represented by X, halogeno groups such as a fluoro group, a chloro group, a bromo group and an iodo group; and C1-6 alkoxy groups such as a methoxy group, an ethoxy group, a n-propoxy group, an i-propoxy group, a n-butoxy group, a s-butoxy group, an i-butoxy group and a t-butoxy group may be preferably exemplified.

As the "C3-8 cycloalkyl group" represented by X, a cyclopropyl group, a cyclobutyl group, a cyclopentyl group, a cyclohexyl group or the like may be exemplified.

As the "C6-10 aryloxy group" represented by X, a phenoxy group, a naphthyloxy group or the like may be exemplified.

As the "5- to 6-membered heteroaryloxy group" represented by X, a pyridyloxy group, a pyrimidyloxy group or the like may be exemplified.

As the substituents on the "C3-8 cycloalkyl group", the "C6-10 aryloxy group" and the "5- to 6-membered heteroaryloxy group" represented by X, halogeno groups such as a fluoro group, a chloro group, a bromo group and an iodo group; C1-6 alkyl groups such as a methyl group, an ethyl group, a n-propyl group, an i-propyl group, a n-butyl group, a s-butyl group, an i-butyl group, a t-butyl group, a n-pentyl group and a n-hexyl group; C1-6 haloalkyl groups such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group and a 2,4,6-trichlorohexyl group; C1-6 alkoxy groups such as a methoxy group, an ethoxy group, a n-propoxy group, an i-propoxy group, a n-butoxy group, a s-butoxy group, an i-butoxy group and a t-butoxy group; and C1-6 haloalkoxy groups such as a 2-chloro-n-propoxy group, a 2,3-dichlorobutoxy group and a trifluoromethoxy group may be preferably exemplified.

The "substituted or unsubstituted amino group" represented by X is a group represented by "$-NR^aR^b$". As $R^a$ and $R^b$ in the formula, each independently, a hydrogen atom, a C1-6 alkyl group, a formyl group, a C1-6 alkylcarbonyl group, a substituted or unsubstituted aminocarbonyl group or the like may be exemplified.

As the "C1-6 alkyl group" represented by each of $R^a$ and $R^b$, the same group as exemplified for $R^2$ and $R^3$ above is exemplified.

As the "C1-6 alkylcarbonyl group" represented by each of $R^a$ and $R^b$, an acetyl group, a propionyl group or the like may be exemplified.

As the "substituted or unsubstituted aminocarbonyl group" represented by each of $R^a$ and $R^b$, an aminocarbonyl group, a methylaminocarbonyl group, an ethylaminocarbonyl group, a dimethylaminocarbonyl group or the like may be exemplified.

As the "substituted or unsubstituted aminocarbonyl group" represented by X, the same group as exemplified for $R^a$ and $R^b$ above is exemplified.

The "substituted or unsubstituted hydrazinyl group" represented by X is a group represented by the formula (a).

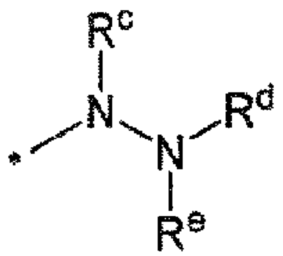

(a)

In the formula (a), * represents a binding position, $R^c$, $R^d$ and $R^e$ each independently represent a hydrogen atom, a C1-6 alkyl group or a substituted or unsubstituted phenylsulfonyl group.

As the "C1-6 alkyl group" represented by each of $R^c$, $R^d$ and $R^e$, the same group as exemplified for X above is exemplified.

As the "substituted phenylsulfonyl group" represented by each of $R^c$, $R^d$ and $R^e$, a paratoluenesulfonyl group

or the like may be exemplified.

[n]

In the formula (I), n represents a number of chemically acceptable Xs, and is an integer of 0 to 4. Xs are the same or different when n is 2 or more.

[A]

In the formula (III), A represents a nitrogen atom or $CR^2$.

As $R^2$ in $CR^2$, the same group as exemplified for $R^2$ above is exemplified.

The salt of each of the compounds (I) to (III) is not particularly limited as long as the salt is agriculturally or horticulturally acceptable. As the salt of each of the compounds (I) to (III), for example, a salt of an inorganic acid such as hydrochloric acid and sulfuric acid; a salt of an organic acid such as acetic acid and lactic acid; a salt of an alkali metal such as lithium, sodium and potassium; a salt of an alkaline earth metal such as calcium and magnesium; a salt of a transition metal such as iron and copper; ammonia; a salt of an organic base such as triethylamine, tributylamine, pyridine and hydrazine; or the like may be exemplified.

The method for producing the compounds (I) to (III) or salts thereof is not particularly limited. For example, the compounds (I) to (III) of the present invention or salts thereof may be obtained by known methods described in Examples, etc. Alternatively, the salts of the compounds (I) to (III) may be obtained by known approaches from the compounds (I) to (III).

The imidazo[1,2-a]pyridine compound of the present invention is excellent in control effect on pests such as various agricultural insect pests and mites affecting the growth of plants.

Also, the imidazo[1,2-a]pyridine compound of the present invention is a highly safe substance because of less phytotoxicity to crops and low toxicity to fishes and warm-blooded animals. Hence, the imidazo[1,2-a]pyridine compound of the present invention is useful as an active ingredient for insecticides or acaracides.

Furthermore, in recent years, many insect pests such as diamondback moth, white-backed plant hopper, leafhopper, and aphid have developed resistance to various existing chemicals, causing problems of insufficient efficacy of these chemicals. Thus, chemicals effective for insect pests of resistant strains have been desired. The imidazo[1,2-a]pyridine compound of the present invention exhibits an excellent control effect not only on sensitive strains but also on insect pests of various resistant strains and even mites of miticide-resistant strains.

The imidazo[1,2-a]pyridine compound of the present invention is excellent in control effect on ectoparasites and endoparasites harmful to humans and animals. Also, the imidazo[1,2-a]pyridine compound of the present invention is a highly safe substance because of low toxicity to fishes and warm-blooded animals. Hence, the imidazo[1,2-a]pyridine compound of the present invention is useful as an active ingredient for ectoparasite and endoparasite control agents.

The imidazo[1,2-a]pyridine compound of the present invention exhibits efficacy at every developmental stage of organisms to be controlled, and exhibits an excellent control effect on, for example, eggs, nymphs, larvae, pupae, and adults of mites, insects, and the like.

[Pest control agent]

The pest control agent of the present invention contains at least one active ingredient selected from the imidazo[1,2-a]pyridine compounds of the present invention. The amount of the imidazo[1,2-a]pyridine compound contained in the pest control agent of the present invention is not particularly limited as long as its pest control effect is exhibited. The pest control agent is an agent controlling pests and includes an insecticide or acaracide, an ectoparasite control agent, or an endoparasite control agent or expellant, or the like.

[Insecticide or acaracide]

The insecticide or acaracide of the present invention contains at least one active ingredient selected from the imidazo[1,2-a]pyridine compounds of the present invention. The amount of the imidazo[1,2-a]pyridine compound contained in the insecticide or acaracide of the present invention is not particularly limited as long as its insecticidal or acaracidal effect is exhibited.

The pest control agent or the insecticide or acaracide of the present invention is preferably used for plants such as cereals; vegetables; root vegetables; tubers and roots; flowers and ornamental plants; fruit trees; ornamental foliage plants and trees of tea, coffee, cacao, and the like; feed crops; lawn grasses; and cotton.

In the application to plants, the pest control agent or the insecticide or acaracide of the present invention may be used for any site such as a leaf, a stem, a stalk, a flower, a bud, a fruit, a seed, a sprout, a root, a tuber, a tuberous

root, a shoot, or a slip.

The pest control agent or the insecticide or acaracide of the present invention is not particularly limited by the species of the plant to which the pest control agent or the insecticide or acaracide is applied. As the plant species, for example, an original species, a variant species, an improved variety, a cultivar, a mutant, a hybrid, a genetically modified organism (GMO) or the like may be exemplified.

The pest control agent of the present invention may be used in seed treatment, foliage application, soil application, submerged application, or the like in order to control various agricultural insect pests and mites.

Specific examples of various agricultural insect pests and mites controllable with the pest control agent of the present invention will be shown below.

(1) Butterflies or moths of the order *Lepidoptera*

(a) moths of the family *Arctiidae,* for example, *Hyphantria cunea* and *Lemyra imparilis;*
(b) moths of the family *Bucculatricidae,* for example, *Bucculatrix pyrivorella;*
(c) moths of the family *Carposinidae,* for example, *Carposina sasakii;*
(d) moths of the family *Crambidae,* for example, *Diaphania indica* and *Diaphania nitidalis* of *Diaphania* spp.; for example, *Ostrinia furnacalis, Ostrinia nubilalis,* and *Ostrinia scapulalis* of *Ostrinia* spp.; and *Chilo suppressalis, Cnaphalocrocis medinalis, Conogethes punctiferalis, Diatraea grandiosella, Glyphodes pyloalis, Hellula undalis,* and *Parapediasia teterrella;*
(e) moths of the family *Gelechiidae,* for example, *Helcystogramma triannulella, Pectinophora gossypiella, Phthorimaea operculella,* and *Sitotroga cerealella;*
(f) moths of the family *Geometridae,* for example, *Ascotis selenaria;*
(g) moths of the family *Gracillariidae,* for example, *Caloptilia theivora, Phyllocnistis citrella,* and *Phyllonorycter ringoniella;*
(h) butterflies of the family *Hesperiidae,* for example, *Parnara guttata;*
(i) moths of the family *Lasiocampidae,* for example, *Malacosoma neustria;*
(j) moths of the family *Lymantriidae,* for example, *Lymantria dispar* and *Lymantria monacha* of *Lymantria* spp.; and *Euproctis pseudoconspersa* and *Orgyia thyellina;*
(k) moths of the family Lyonetiidae, for example, *Lyonetia clerkella* and *Lyonetia prunifoliella malinella* of *Lyonetia* spp. ;
(l) moths of the family *Noctuidae,* for example, *Spodoptera depravata, Spodoptera eridania, Spodoptera exigua, Spodoptera frugiperda, Spodoptera littoralis,* and *Spodoptera litura* of *Spodoptera* spp.; for example, *Autographa gamma* and *Autographa nigrisigna* of *Autographa* spp.; for example, *Agrotis ipsilon* and *Agrotis segetum* of *Agrotis* spp.; for example, *Helicoverpa armigera, Helicoverpa assulta,* and *Helicoverpa zea* of *Helicoverpa* spp.; for example, *Heliothis armigera* and *Heliothis virescens* of *Heliothis* spp.; and *Aedia leucomelas, Ctenoplusia agnata, Eudocima tyrannus, Mamestra brassicae, Mythimna separata, Naranga aenescens, Panolis japonica, Peridroma saucia, Pseudoplusia includens,* and *Trichoplusia ni;*
(m) moths of the family *Nolidae,* for example, *Earias insulana;*
(n) butterflies of the family *Pieridae,* for example, *Pieris brassicae* and *Pieris rapae crucivora* of *Pieris* spp.;
(o) moths of the family *Plutellidae,* for example, *Acrolepiopsis sapporensis* and *Acrolepiopsis suzukiella* of *Acrolepiopsis* spp.; and *Plutella xylostella;*
(p) moths of the family *Pyralidae,* for example, *Cadra cautella, Elasmopalpus lignosellus, Etiella zinckenella,* and *Galleria mellonella;*
(q) moths of the family *Sphingidae,* for example, *Manduca quinquemaculata* and *Manduca sexta* of *Manduca* spp.;
(r) moths of the family *Stathmopodidae,* for example, *Stathmopoda masinissa;*
(s) moths of the family *Tineidae,* for example, *Tinea transducers;*
(t) moths of the family *Tortricidae,* for example, *Adoxophyes honmai* and *Adoxophyes orana* of *Adoxophyes* spp.; for example, *Archips breviplicanus* and *Archips fuscocupreanus* of *Archips* spp.; and *Choristoneura fumiferana, Cydia pomonella, Eupoecilia ambiguella, Grapholitha molesta, Homona magnanima, Leguminivora glycinivorella, Lobesia botrana, Matsumuraeses phaseoli, Pandemis heparana,* and *Sparganothis pilleriana;*
(u) moths of the family *Yponomeutidae,* for example, *Argyresthia conjugella.*

(2) Insect pests of the order *Thysanoptera*

(a) insect pests of the family *Phlaeothripidae,* for example, *Ponticulothrips diospyrosi;*
(b) insect pests of the family *Thripidae,* for example, *Frankliniella intonsa* and *Frankliniella occidentalis* of *Frankliniella* spp.; for example, *Thrips palmi* and *Thrips tabaci* of *Thrips* spp.; and *Heliothrips haemorrhoidalis* and

*Scirtothrips dorsalis.*

(3) Insect pests of the order *Hemiptera*

(A) the suborder *Archaeorrhyncha*

(a) insect pests of the family *Delphacidae,* for example, *Laodelphax striatella, Nilaparvata lugens, Perkinsiella saccharicida,* and *Sogatella furcifera.*

(B) the suborder *Clypeorrhyncha*

(a) insect pests of the family *Cicadellidae,* for example, *Empoasca fabae, Empoasca nipponica, Empoasca onukii,* and *Empoasca sakaii* of *Empoasca* spp.; and *Arboridia apicalis, Balclutha saltuella, Epiacanthus stramineus, Macrosteles striifrons,* and *Nephotettix cinctinceps.*

(C) the suborder *Heteroptera*

(a) insect pests of the family *Alydidae,* for example, *Riptortus clavatus;*
(b) insect pests of the family *Coreidae,* for example, *Cletus punctiger* and *Leptocorisa chinensis;*
(c) insect pests of the family *Lygaeidae,* for example, *Blissus leucopterus, Cavelerius saccharivorus,* and *Togo hemipterus;*
(d) insect pests of the family *Miridae,* for example, *Halticus insularis, Lygus lineolaris, Psuedatomoscelis seriatus, Stenodema sibiricum, Stenotus rubrovittatus,* and *Trigonotylus caelestialium;*
(e) insect pests of the family *Pentatomidae,* for example, *Nezara antennata* and *Nezara viridula* of *Nezara* spp.; for example, *Eysarcoris aeneus, Eysarcoris lewisi,* and *Eysarcoris ventralis* of *Eysarcoris* spp.; and *Dolycoris baccarum, Eurydema rugosum, Glaucias subpunctatus, Halyomorpha halys, Piezodorus hybneri, Plautia crossota,* and *Scotinophora lurida;*
(f) insect pests of the family *Pyrrhocoridae,* for example, *Dysdercus cingulatus;*
(g) insect pests of the family *Rhopalidae,* for example, *Rhopalus msculatus;*
(h) insect pests of the family *Scutelleridae,* for example, *Eurygaster integriceps;*
(i) insect pests of the family *Tingidae,* for example, *Stephanitis nashi.*

(D) the suborder *Sternorrhyncha*

(a) insect pests of the family *Adelgidae,* for example, *Adelges laricis;*
(b) insect pests of the family *Aleyrodidae,* for example, *Bemisia argentifolii* and *Bemisia tabaci* of *Bemisia* spp.; and *Aleurocanthus spiniferus, Dialeurodes citri,* and *Trialeurodes vaporariorum;*
(c) insect pests of the family *Aphididae,* for example, Aphis *craccivora, Aphis fabae, Aphis forbesi, Aphis gossypii, Aphis pomi, Aphis sambuci,* and *Aphis spiraecola* of *Aphis* spp.; for example, *Rhopalosiphum maidis* and *Rhopalosiphum padi* of *Rhopalosiphum* spp.; for example, *Dysaphis plantaginea* and *Dysaphis radicola* of *Dysaphis* spp.; for example, *Macrosiphum avenae* and *Macrosiphum euphorbiae* of *Macrosiphum* spp.; for example, *Myzus cerasi, Myzus persicae,* and *Myzus varians* of *Myzus* spp.; and *Acyrthosiphon pisum, Aulacorthum solani, Brachycaudus helichrysi, Brevicoryne brassicae, Chaetosiphon fragaefolii, Hyalopterus pruni, Hyperomyzus lactucae, Lipaphis erysimi, Megoura viciae, Metopolophium dirhodum, Masonovia ribis-nigri, Phorodon humuli, Schizaphis graminum, Sitobion avenae,* and *Toxoptera aurantii;*
(d) insect pests of the family *Coccidae,* for example, *Ceroplastes ceriferus* and *Ceroplastes rubens* of *Ceroplastes* spp. ;
(e) insect pests of the family *Diaspididae, Pseudaulacaspis pentagona* and *Pseudaulacaspis prunicola* of *Pseudaulacaspis* spp.; for example, *Unaspis euonymi* and *Unaspis yanonensis* of *Unaspis* spp.; and *Aonidiella aurantii, Comstockaspis perniciosa, Fiorinia theae,* and *Pseudaonidia paeoniae;*
(f) insect pests of the family *Margarodidae,* for example, *Drosicha corpulenta* and *Icerya purchasi;*
(g) insect pests of the family *Phylloxeridae,* for example, *Viteus vitifolii;*
(h) insect pests of the family *Pseudococcidae,* for example, *Planococcus citri* and *Planococcus kuraunhiae* of *Planococcus* spp.; and *Phenacoccus solani* and *Pseudococcus comstocki;*
(i) insect pests of the family *Psyllidae,* for example, *Psylla mali* and *Psylla pyrisuga* of *Psylla* spp.; and *Diaphorina citri.*

(4) Insect pests of the suborder *Polyphaga*

(a) insect pests of the family *Anobiidae,* for example, *Lasioderma serricorne;*
(b) insect pests of the family *Attelabidae,* for example, *Byctiscus betulae* and *Rhynchites heros;*
(c) insect pests of the family *Bostrichidae,* for example, *Lyctus brunneus;*
(d) insect pests of the family *Brentidae,* for example, *Cylas formicarius;*
(e) insect pests of the family *Buprestidae,* for example, *Agrilus sinuatus;*
(f) insect pests of the family *Cerambycidae,* for example, *Anoplophora malasiaca, Monochamus alternatus, Psacothea hilaris,* and *Xylotrechus pyrrhoderus;*
(g) insect pests of the family *Chrysomelidae,* for example, *Bruchus pisorum* and *Bruchus rufimanus* of *Bruchus* spp.; for example, *Diabrotica barberi, Diabrotica undecimpunctata,* and *Diabrotica virgifera* of *Diabrotica* spp.; for example, *Phyllotreta nemorum* and *Phyllotreta striolata* of *Phyllotreta* spp.; and *Aulacophora femoralis, Callosobruchus chinensis, Cassida nebulosa, Chaetocnema concinna, Leptinotarsa decemlineata, Oulema oryzae,* and *Psylliodes angusticollis;*
(h) insect pests of the family *Coccinellidae,* for example, *Epilachna varivestis* and *Epilachna vigintioctopunctata* of *Epilachna* spp.;
(i) insect pests of the family *Curculionidae,* for example, *Anthonomus grandis* and *Anthonomus pomorum* of *Anthonomus* spp.; for example, *Sitophilus granarius* and *Sitophilus zeamais* of *Sitophilus* spp.; and *Echinocnemus squameus, Euscepes postfasciatus, Hylobius abietis, Hypera postica, Lissohoptrus oryzophilus, Otiorhynchus sulcatus, Sitona lineatus,* and *Sphenophorus venatus;*
(j) insect pests of the family *Elateridae,* for example, *Melanotus fortnumi* and *Melanotus tamsuyensis* of *Melanotus* spp. ;
(k) insect pests of the family *Nitidulidae,* for example, *Epuraea domina;*
(l) insect pests of the family *Scarabaeidae,* for example, *Anomala cuprea* and *Anomala rufocuprea* of *Anomala* spp.; and *Cetonia aurata, Gametis jucunda, Heptophylla picea, Melolontha melolontha,* and *Popillia japonica;*
(m) insect pests of the family *Scolytidae,* for example, *Ips typographus;*
(n) insect pests of the family *Staphylinidae,* for example, *Paederus fuscipes;*
(o) insect pests of the family *Tenebrionidae,* for example, *Tenebrio molitor* and *Tribolium castaneum;*
(p) insect pests of the family *Trogossitidae,* for example, *Tenebroides mauritanicus.*

(5) Insect pests of the order *Diptera*

(A) the suborder *Brachycera*

(a) insect pests of the family *Agromyzidae,* for example, *Liriomyza bryoniae, Liriomyza chinensis, Liriomyza sativae,* and *Liriomyza trifolii* of *Liriomyza* spp.; and *Chromatomyia horticola* and *Agromyza oryzae;*
(b) insect pests of the family *Anthomyiidae,* for example, *Delia platura* and *Delia radicum* of *Delia* spp.; and *Pegomya cunicularia;*
(c) insect pests of the family *Drosophilidae,* for example, *Drosophila melanogaster* and *Drosophila suzukii* of *Drosophila* spp.;
(d) insect pests of the family *Ephydridae,* for example, *Hydrellia griseola;*
(e) insect pests of the family *Psilidae,* for example, *Psila rosae;*
(f) insect pests of the family *Tephritidae,* for example, *Bactrocera cucurbitae* and *Bactrocera dorsalis* of *Bactrocera* spp.; for example, *Rhagoletis cerasi* and *Rhagoletis pomonella* of *Rhagoletis* spp.; and *Ceratitis capitata* and *Dacus oleae.*

(B) the suborder Nematocera

(a) insect pests of the family *Cecidomyiidae,* for example, *Asphondylia yushimai, Contarinia sorghicola, Mayetiola destructor,* and *Sitodiplosis mosellana.*

(6) Insect pests of the order *Orthoptera*

(a) insect pests of the family *Acrididae,* for example, *Schistocerca americana* and *Schistocerca gregaria* of *Schistocerca* spp.; and *Chortoicetes terminifera, Dociostaurus maroccanus, Locusta migratoria, Locustana pardalina, Nomadacris septemfasciata,* and *Oxya yezoensis;*
(b) insect pests of the family *Gryllidae,* for example, *Acheta domestica* and *Teleogryllus emma;*
(c) insect pests of the family *Gryllotalpidae,* for example, *Gryllotalpa orientalis;*
(d) insect pests of the family *Tettigoniidae,* for example, *Tachycines asynamorus.*

(7) *Acari*

(A) *Acaridida* of the order *Astigmata*

(a) mites of the family *Acaridae,* for example, *Rhizoglyphus echinopus* and *Rhizoglyphus robini* of *Rhizoglyphus* spp.; for example, *Tyrophagus neiswanderi, Tyrophagus perniciosus, Tyrophagus putrescentiae,* and *Tyrophagus similis* of *Tyrophagus* spp.; and *Acarus siro, Aleuroglyphus ovatus,* and *Mycetoglyphus fungivorus;*

(B) *Actinedida* of the order *Prostigmata*

(a) mites of the family *Tetranychidae,* for example, *Bryobia praetiosa* and *Bryobia rubrioculus* of *Bryobia* spp.; for example, *Eotetranychus asiaticus, Eotetranychus boreus, Eotetranychus celtis, Eotetranychus geniculatus, Eotetranychus kankitus, Eotetranychus pruni, Eotetranychus shii, Eotetranychus smithi, Eotetranychus suginamensis,* and *Eotetranychus uncatus* of *Eotetranychus* spp.; for example, *Oligonychus hondoensis, Oligonychus ilicis, Oligonychus karamatus, Oligonychus mangiferus, Oligonychus orthius, Oligonychus perseae, Oligonychus pustulosus, Oligonychus shinkajii,* and *Oligonychus ununguis* of *Oligonychus* spp.; for example, *Panonychus citri, Panonychus mori,* and *Panonychus ulmi* of *Panonychus* spp.; for example, *Tetranychus cinnabarinus, Tetranychus kanzawai, Tetranychus ludeni, Tetranychus quercivorus, Tetranychus phaselus, Tetranychus urticae, Tetranychus viennensis,* and *Tetranychus evansi* of *Tetranychus* spp.; for example, *Aponychus corpuzae* and *Aponychus firmianae* of *Aponychus* spp.; for example, *Sasanychus akitanus* and *Sasanychus pusillus* of *Sasanychus* spp.; for example, *Schizotetranychus celarius, Schizotetranychus longus, Schizotetranychus miscanthi, Schizotetranychus recki,* and *Schizotetranychus schizopus* of *Schizotetranychus* spp.; and *Tetranychina harti, Tuckerella pavoniformis,* and *Yezonychus sapporensis;*
(b) mites of the family *Tenuipalpidae,* for example, *Brevipalpus lewisi, Brevipalpus obovatus, Brevipalpus phoenicis, Brevipalpus russulus,* and *Brevipalpus californicus* of *Brevipalpus* spp.; for example, *Tenuipalpus pacificus* and *Tenuipalpus zhizhilashviliae* of *Tenuipalpus* spp.; and *Dolichotetranychus floridanus;*
(c) mites of the family *Eriophyidae,* for example, *Aceria diospyri, Aceria ficus, Aceria japonica, Aceria kuko, Aceria paradianthi, Aceria tiyingi, Aceria tulipae,* and *Aceria zoysiea* of *Aceria* spp.; for example, *Eriophyes chibaensis* and *Eriophyes emarginatae* of *Eriophyes* spp.; for example, *Aculops lycopersici* and *Aculops pelekassi* of *Aculops* spp.; for example, *Aculus fockeui* and *Aculus schlechtendali* of *Aculus* spp.; and *Acaphylla theavagrans, Calacarus carinatus, Colomerus vitis, Calepitrimerus vitis, Epitrimerus pyri, Paraphytoptus kikus, Paracalacarus podocarpi,* and *Phyllocotruta citri;*
(d) mites of the family *Tarsonemidae,* for example, *Tarsonemus bilobatus* and *Tarsonemus waitei* of *Tarsonemus* spp.; and *Phytonemus pallidus* and *Polyphagotarsonemus latus;*
(e) mites of the family Penthaleidae, for example, *Penthaleus erythrocephalus* and *Penthaleus major* of *Penthaleus* spp.

The pest control agent of the present invention may be used as a mixture or in combination with another active ingredient such as a fungicide, an insecticide or acaracide, a nematicide, or a pesticide for soil insect pests; a plant regulating agent, a synergist, a fertilizer, a soil improvement agent, animal feed, or the like.

The combination of the compound of the present invention with another active ingredient may be expected to have synergistic effects on insecticidal, acaracidal, or nematicidal activity. The synergistic effects may be confirmed by the equation of Colby (Colby. S.R.; Calculating Synergistic and Antagonistic Responses of Herbicide Combinations; Weeds 15, p. 20-22, 1967) according to a standard method.

Specific examples of the insecticide or acaracide, the nematicide, the pesticide for soil insect pests, the anthelmintic agent, and the like that may be used as a mixture or in combination with the pest control agent of the present invention will be shown below.

(1) Acetylcholinesterase inhibitors:

(a) carbamate-based: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylylcarb, fenothiocarb, MIPC, MPMC, MTMC, aldoxycarb, allyxycarb, aminocarb, bufencarb, cloethocarb, metam sodium, and promecarb;
(b) organophosphorus-based: acephate, azamethiphos, azinphos-ethyl, azinphos-methyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, bromophos-ethyl, BRP, carbophenothion, cyanofenphos, CYAP, demeton-S-methyl sulfone, dialifos, dichlofenthion, dioxabenzofos, etrimfos, fensulfothion, flupyrazofos, fonofos, formothion, fosmethilan, isazofos, iodofenphos, methacrifos, pirimiphos-ethyl, phosphocarb, propaphos, prothoate, and sulprofos.

(2) GABAergic chloride ion channel antagonists: acetoprole, chlordene, endosulfan, ethiprole, fipronil, pyrafluprole, pyriprole, camphechlor, heptachlor, and dienochlor.
(3) Sodium channel modulators: acrinathrin, d-cis-trans allethrin, d-trans allethrin, bifenthrin, bioallethrin, bioallethrin S-cyclopentyl isomer, bioresmethrin, cycloprothrin, cyfluthrin,β-cyfluthrin, cyhalothrin, X-cyhalothrin, γ-cyhalothrin, cypermethrin, α-cypermethrin, β-cypermethrin, θ-cypermethrin, ζ-cypermethrin, cyphenothrin [(1R)-trans isomer], deltamethrin, empenthrin [(EZ)-(1R)-Isomer], esfenvalerate, etofenprox, fenpropathrin, fenvalerate, flucythrinate, flumethrin, τ-fluvalinate, halfenprox, imiprothrin, kadethrin, permethrin, phenothrin [(1R)-trans isomer], prallethrin, pyrethrum, resmethrin, silafluofen, tefluthrin, tetramethrin [(1R)-isomer], tralomethrin, transfluthrin, allethrin, pyrethrins, pyrethrin I, pyrethrin II, profluthrin, dimefluthrin, bioethanomethrin, biopermethrin, transpermethrin, fenfluthrin, fenpirithrin, flubrocythrinate, flufenprox, metofluthrin, protrifenbute, pyresmethrin, and terallethrin.
(4) Nicotinic acetylcholine receptor agonists: acetamiprid, clothianidin, dinotefuran, imidacloprid, nitenpyram, nithiazine, thiacloprid, thiamethoxam, sulfoxaflor, nicotine, flupyradifurone, and flupyrimine.
(5) Nicotinic acetylcholine receptor allosteric modulators: spinetoram and spinosad.
(6) Chloride channel activators: abamectin, emamectin - benzoate, lepimectin, milbemectin, ivermectin, selamectin, doramectin, eprinomectin, moxidectin, milbemycin, milbemycin oxime, and nemadectin.
(7) Juvenile hormone-like substances: hydroprene, kinoprene, methoprene, fenoxycarb, pyriproxyfen, diofenolan, epofenonane, and triprene.
(8) Other nonspecific inhibitors: methyl bromide, chloropicrin, sulfuryl fluoride, borax, and tartar emetic.
(9) Homoptera selective feeding inhibitors: flonicamid, pymetrozine, and pyrifluquinazon.
(10) Mite growth inhibitors: clofentezine, diflovidazin, hexythiazox, and etoxazole.
(11) Insect midgut inner membrane disrupting agents derived from microorganisms: *bacillus thuringiensis* subsp. *Israelensis, bacillus sphaericus, bacillus thuringiensis* subsp. *aizawai, bacillus thuringiensis* subsp. *kurstaki, bacillus thuringiensis* subsp. *tenebrionis,* and Bt crop proteins: Cry1Ab, Cry1Ac, Cry1Fa, Cry1A.105, Cry2Ab, Vip3A, mCry3A, Cry3Ab, Cry3Bb, and Cry34Ab1, Cry35Ab1.
(12) Mitochondrial ATP biosynthetic enzyme inhibitors: diafenthiuron, azocyclotin, cyhexatin, fenbutatin oxide, propargite, and tetradifon.
(13) Oxidative phosphorylation uncouplers: chlorfenapyr, sulfluramid, DNOC, binapacryl, dinobuton, and dinocap.
(14) Nicotinic acetylcholine receptor channel blockers: bensultap, cartap hydrochloride, nereistoxin, thiosultap-sodium, and thiocyclam.
(15) Chitin synthesis inhibitors: bistrifluron, chlorfluazuron, diflubenzuron, flucycloxuron, flufenoxuron, hexaflumuron, lufenuron, novaluron, noviflumuron, teflubenzuron, triflumuron, buprofezin, and fluazuron.
(16) Diptera molting disrupting agents: cyromazine.
(17) Molting hormone receptor agonists: chromafenozide, halofenozide, methoxyfenozide, and tebufenozide.
(18) Octopamine receptor agonists: amitraz, demiditraz, and chlordimeform.
(19) Mitochondrial electron transport system complex III inhibitors: acequinocyl, fluacrypyrim, and hydramethylnon.
(20) Mitochondrial electron transport system complex I inhibitors: fenazaquin, fenpyroximate, pyrimidifen, pyridaben, tebufenpyrad, tolfenpyrad, and rotenone.
(21) Voltage-gated sodium channel blockers: indoxacarb and metaflumizone.
(22) Acetyl CoA carboxylase inhibitors: spirodiclofen, spiromesifen, and spirotetramat.
(23) Mitochondrial electron transport system complex IV inhibitors: aluminum phosphide, calcium phosphide, phosphine, zinc phosphide, and cyanide.
(24) Mitochondrial electron transport system complex II inhibitors: cyenopyrafen, cyflumetofen, and pyflubumide.
(25) Ryanodine receptor modulators: chlorantraniliprole, cyantraniliprole, flubendiamide, cyclaniliprole, and tetraniliprole.
(26) Mixed function oxidase inhibitor compounds: piperonyl butoxide.
(27) Latrophilin receptor agonists: depsipeptide, cyclodepsipeptide, 24 membered cyclodepsipeptide, and emodepside.

(28) Other agents (based on an unknown mechanism of action): azadirachtin, benzoximate, bifenazate, bromopropylate, quinomethionate, cryolite, dicofol, pyridalyl, benclothiaz, sulfur, amidoflumet, 1,3-dichloropropene, DCIP, phenisobromolate, benzomate, metaldehyde, chlorobenzilate, clothiazoben, dicyclanil, fenoxacrim, fentrifanil, flubenzimine, fluphenazine, gossyplure, japonilure, metoxadiazone, oil, potassium oleate, tetrasul, triarathene, afidopyropen, flometoquin, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazole-3-yl]-2-(1H-1,2,4-triazole-1-yl)benzonitrile (CAS: 943137-49-3), broflanilide, and other m-diamides.
(29) Anthelmintic agents:

(a) benzimidazole-based: fenbendazole, albendazole, triclabendazole, oxibendazole, mebendazole, oxfendazole, parbendazole, flubendazole, febantel, netobimin, thiophanate, thiabendazole, and cambendazole;
(b) salicylanilide-based: closantel, oxyclozanide, rafoxanide, and niclosamide;
(c) substituted phenol-based: nitroxinil and nitroscanate;
(d) pyrimidine-based: pyrantel and morantel;
(e) imidazothiazole-based: levamisole and tetramisole;
(f) tetrahydropyrimidine-based: praziquantel and epsiprantel;
(g) other anthelmintic agents: cyclodiene, ryania, clorsulon, metronidazole, demiditraz, piperazine, diethylcarbamazine, dichlorophen, monepantel, tribendimidine, amidantel, thiacetarsamide, melarsomine, and arsenamide.

Specific examples of the fungicide that may be used as a mixture or in combination with the pest control agent of the present invention will be shown below.

(1) Nucleic acid biosynthesis inhibitors:

(a) RNA polymerase I inhibitors: benalaxyl, benalaxyl-M, furalaxyl, metalaxyl, metalaxyl-M, oxadixyl, clozylacon, and ofurace;
(b) adenosine deaminase inhibitors: bupirimate, dimethirimol, and ethirimol;
(c) DNA/RNA synthesis inhibitors: hymexazol and octhilinone;
(d) DNA topoisomerase II inhibitors: oxolinic acid.

(2) Mitotic inhibitors and cell division inhibitors:

(a) β-tubulin polymerization inhibitors: benomyl, carbendazim, chlorfenazole, fuberidazole, thiabendazole, thiophanate, thiophanate-methyl, diethofencarb, zoxamide, and ethaboxam;
(b) cell division inhibitors: pencycuron;
(c) spectrin-like protein delocalization inhibitors: fluopicolide.

(3) Respiration inhibitors:

(a) complex I NADH oxidation-reduction enzyme inhibitors: diflumetorim and tolfenpyrad;
(b) complex II succinate dehydrogenase inhibitors: benodanil, flutolanil, mepronil, isofetamid, fluopyram, fenfuram, furmecyclox, carboxin, oxycarboxin, thifluzamide, benzovindiflupyr, bixafen, fluxapyroxad, furametpyr, isopyrazam, penflufen, penthiopyrad, sedaxane, and boscalid;
(c) complex III ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, and pyribencarb;
(d) complex III ubiquinol reductase Qi inhibitors: cyazofamid, and amisulbrom;
(e) oxidative phosphorylation uncoupling agents: binapacryl, meptyldinocap, dinocap, fluazinam, and ferimzone;
(f) oxidative phosphorylation inhibitors (ATP synthase inhibitors): fentin acetate, fentin chloride, and fentin hydroxide;
(g) ATP production inhibitors: silthiofam;
(h) complex III: Qx (unknown) inhibitor of cytochrome bc1 (ubiquinone reductase): ametoctradin.

(4) Amino acid and protein synthesis inhibitors

(a) methionine biosynthesis inhibitors: andoprim, cyprodinil, mepanipyrim, and pyrimethanil;

(b) protein synthesis inhibitors: blasticidin-S, kasugamycin, kasugamycin hydrochloride, streptomycin, and oxytetracycline.

(5) Signal transduction inhibitors:

(a) signal transduction inhibitors: quinoxyfen and proquinazid;
(b) MAP/histidine kinase inhibitors in osmotic signal transduction: fenpiclonil, fludioxonil, chlozolinate, iprodione, procymidone, and vinclozolin.

(6) Lipid and cell membrane synthesis inhibitors:

(a) phospholipid biosynthesis, methyltransferase inhibitors: edifenphos, iprobenfos, pyrazophos, and isoprothiolane;
(b) lipid peroxidation agents: biphenyl, chloroneb, dichloran, quintozene, tecnazene, tolclofos-methyl, and etridiazole;
(c) agents that act on cell membranes: iodocarb, propamocarb, propamocarb-hydrochloride, propamocarb-fosetylate, and prothiocarb;
(d) microorganisms that disrupt cell membranes of pathogens: *bacillus subtilis, bacillus subtilis* strain QST713, *bacillus subtilis* strain FZB24, *bacillus subtilis* strain MBI600, and *bacillus subtilis* strain D747;
(e) agents that disrupt cell membranes: *melaleuca alternifolia* (tea tree) extract.

(7) Cell membrane sterol biosynthesis inhibitors:

(a) C14-demethylation inhibitors in sterol biosynthesis: triforine, pyrifenox, pyrisoxazole, fenarimol, flurprimidol, nuarimol, imazalil, imazalil-sulfate, oxpoconazole, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, quinconazole, simeconazole, tebuconazole, tetraconazole, triadimefon, triadimenol, triticonazole, prothioconazole, and voriconazole;
(b) $\Delta 14$ reductase and sterol $\Delta 8 \rightarrow \Delta 7$-isomerase inhibitors in sterol biosynthesis: aldimorph, dodemorph, dodemorph acetate, fenpropimorph, tridemorph, fenpropidin, piperalin, and spiroxamine;
(c) 3-keto reductase inhibitors in C4-demethylation in the sterol biosynthesis system: fenhexamid and fenpyrazamine;
(d) squalene epoxidase inhibitors in the sterol biosynthesis system: pyributicarb, naftifine, and terbinafine.

(8) Cell wall synthesis inhibitors

(a) trehalase inhibitors: validamycin;
(b) chitin synthase inhibitors: polyoxins and polyoxorim;
(c) cellulose synthase inhibitors: dimethomorph, flumorph, pyrimorph, benthiavalicarb, iprovalicarb, tolprocarb, valifenalate, and mandipropamid.

(9) Melanin biosynthesis inhibitors

(a) melanin biosynthesis reductase inhibitors: fthalide, pyroquilon, and tricyclazole;
(b) melanin biosynthesis anhydrase inhibitors: carpropamid, diclocymet, and fenoxanil.

(10) Host plant resistance inducers:

(a) agents that act on salicylic acid synthesis pathway: acibenzolar-S-methyl;
(b) others: probenazole, tiadinil, isotianil, laminarin, and reynoutria sachalinensis extract.

(11) Agents with unknown mode of action: cymoxanil, fosetyl-aluminum, phosphoric acid (phosphate), tecloftalam, triazoxide, flusulfamide, diclomezine, methasulfocarb, cyflufenamid, metrafenone, pyriofenone, dodine, dodine free base, and flutianil.
(12) Agents having multiple active sites: copper (copper salt), bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, iminoctadine triacetate, iminoctadine trialbesilate, anilazine, dithianon, quinomethionate, and fluoroimide.

(13) Other agents: DBEDC, fluorofolpet, guazatine acetate, bis(8-quinolinolato)copper(II), propamidine, chloropicrin, cyprofuram, agrobacterium, bethoxazin, diphenylamine, methyl isothiocyanate (MITC), mildiomycin, capsaicin, cufraneb, cyprosulfamide, dazomet, debacarb, dichlorophen, difenzoquat, difenzoquat methylsulfonate flumetover, fosetyl-calcium, fosetyl-sodium, irumamycin, natamycin, nitrothal isopropyl, oxamocarb, propamocin-sodium, pyrrolnitrin, tebufloquin, tolnifanide, zarilamide, Algophase, Amicarthiazol, Oxathiapiprolin, metiram zinc, benthiazole, trichlamide, uniconazole, mildiomycin, Oxyfenthiin, and picarbutrazox.

Specific examples of the plant regulating agent that may be used as a mixture or in combination with the pest control agent of the present invention will be shown below.

Abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropane, N-acetyl aminoethoxyvinyl glycine (also called aviglycine), aminooxyacetate, silver nitrate, cobalt chloride, IAA, 4-CPA, cloprop, 2,4-D, MCPB, indole-3-butyrate, dichlorprop, phenothiol, 1-naphthyl acetamide, ethychlozate, cloxyfonac, maleic acid hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyladenine, and 5-amino levulinic acid.

[Ectoparasite control agent]

The ectoparasite control agent of the present invention contains at least one active ingredient selected from the imidazo[1,2-a]pyridine compounds of the present invention. The amount of the imidazo[1,2-a]pyridine compound contained in the ectoparasite control agent of the present invention is not particularly limited as long as its ectoparasite control effect is exhibited.

As the host animal to be treated with the ectoparasite control agent of the present invention, a warm-blooded animal such as a human and a livestock mammal (e.g., a cow, a horse, a pig, sheep, and a goat), a laboratory animal (e.g., a mouse, a rat, and a sand rat), a pet animal (e.g., a hamster, a guinea pig, a dog, a cat, a horse, a squirrel, a rabbit, and a ferret), wild and zoo mammals (e.g., a monkey, a fox, a deer, and a buffalo), a fowl (e.g., a turkey, a duck, a chicken, a quail, and a goose), and a pet bird (e.g., a pigeon, a parrot, a myna bird, a Java sparrow, a parakeet, a Bengalese finch, and a canary bird); and fishes such as salmon, trout, and koi may be exemplified. In addition, a bee, a stag beetle and a beetle may be exemplified.

The ectoparasite control agent of the present invention may be applied by a known veterinary approach (local, oral, parenteral or subcutaneous administration). As the method therefor, a method of orally administering tablets, capsules, feed or the like containing the ectoparasite control agent to animals; a method of administering the ectoparasite control agent through dipping liquids, suppositories, injection (intramuscular, subcutaneous, intravenous, or intraperitoneal injection, etc.) or the like to animals; a method of locally administering oily or aqueous liquid formulations by spraying, pour-on, spot-on or the like; and a method of kneading the ectoparasite control agent into a resin, shaping the kneaded product into a suitable shape such as a collar or an ear tag, and attaching it to animals for local administration; or the like may be exemplified.

Ectoparasites parasitize the inside or the body surface of host animals, particularly, warm-blooded animals. Specifically, the ectoparasites parasitize the backs, armpits, lower abdomens, inner thighs, or the like of host animals and live by obtaining nutrients such as blood or dandruff from the animals. As the ectoparasite, mites, lice, fleas, a mosquito, a stable fly, a flesh fly or the like may be exemplified. Specific examples of the ectoparasite controllable with the ectoparasite control agent of the present invention will be shown below.

(1) *Acari*

mites of the family *Dermanyssidae,* mites of the family *Macronyssidae,* mites of the family *Laelapidae,* mites of the family *Varroidae,* mites of the family *Argasidae,* mites of the family *Ixodidae,* mites of the family *Psoroptidae,* mites of the family *Sarcoptidae,* mites of the family *Knemidokoptidae,* mites of the family *Demodixidae,* mites of the family *Trombiculidae,* and insect parasitic mites such as mites of the family *Canestriniidae.*

(2) The order *Phthiraptera*

lice of the family *Haematopinidae,* lice of the family *Linognathidae,* bird lice of the family *Menoponidae,* bird lice of the family *Philopteridae,* and bird lice of the family *Trichodectidae.*

(3) The order *Siphonaptera*

fleas of the family *Pulicidae,* for example, *Ctenocephalides canis* and *Ctenocephalides felis* of *Ctenocephalides* spp.; fleas of the family *Tungidae,* fleas of the family *Ceratophyllidae,* and fleas of the family *Leptopsyllidae.*

(4) The order *Hemiptera*

(5) Insect pests of the order *Diptera*

mosquitos of the family *Culicidae,* black flies of the family *Simuliidae,* biting midges of the family *Ceratopogonidae,* horseflies of the family *Tabanidae,* flies of the family *Muscidae,* tsetse flies of the family *Glossinidae,* flesh flies of the family *Sarcophagidae,* flies of the family *Hippoboscidae,* flies of the family *Calliphoridae,* and flies of the family *Oestridae.*

[Endoparasite control agent or expellant]

The endoparasite control agent or expellant of the present invention contains at least one active ingredient selected from the imidazo[1,2-a]pyridine compounds of the present invention. The amount of the imidazo[1,2-a]pyridine compound contained in the endoparasite control agent or expellant of the present invention is not particularly limited as long as its endoparasite control effect is exhibited.

The parasite targeted by the endoparasite control agent or expellant of the present invention parasitizes the inside of host animals, particularly, warm-blooded animals or fishes (endoparasite). As the host animal for which the endoparasite control agent or expellant of the present invention is effective, a warm-blooded animal such as a human and a livestock mammal (e.g., a cow, a horse, a pig, sheep, and a goat), a laboratory animal (e.g., a mouse, a rat, and a sand rat), a pet animal (e.g., a hamster, a guinea pig, a dog, a cat, a horse, a squirrel, a rabbit, and a ferret), wild and zoo mammals (e.g., a monkey, a fox, a deer, and a buffalo), a fowl (e.g., a turkey, a duck, a chicken, a quail, and a goose), and a pet bird (e.g., a pigeon, a parrot, a myna bird, a Java sparrow, a parakeet, a Bengalese finch, and a canary bird); and fishes such as salmon, trout, and koi may be exemplified. Parasitic diseases mediated by parasites may be prevented or treated by controlling and expelling the parasites.

As the parasite to be controlled or expelled, the following may be exemplified.

(1) Nematodes of the order *Dioctophymatida*

(a) kidney worms of the family *Dioctophymatidae,* for example, *Dioctophyma renale* of *Dioctophyma* spp.;

(b) kidney worms of the family *Soboliphymatidae,* for example, *Soboliphyme abei* and *Soboliphyme baturini* of *Soboliphyme* spp.

(2) Nematodes of the order *Trichocephalida*

(a) trichinae of the family *Trichinellidae,* for example, *Trichinella spiralis* of *Trichinella* spp.;

(b) whipworms of the family *Trichuridae,* for example, *Capillaria annulata, Capillaria contorts, Capillaria hepatica, Capillaria perforans, Capillaria plica,* and *Capillaria suis* of *Capillaria* spp.; and *Trichuris vulpis, Trichuris discolor, Trichuris ovis, Trichuris skrjabini,* and *Trichuris suis* of *Trichuris* spp.

(3) Nematodes of the order *Rhabditida* threadworms of the family *Strongyloididae,* for example, *Strongyloides papillosus, Strongyloides planiceps, Strongyloides ransoms, Strongyloides suis, Strongyloides stercoralis, Strongyloides tumefaciens,* and *Strongyloides ratti* of *Strongyloides* spp.

(4) Nematodes of the order *Strongylida*

hookworms of the family *Ancylostomatidae,* for example, *Ancylostoma braziliense, Ancylostoma caninum, Ancylostoma duodenale,* and *Ancylostoma tubaeforme* of *Ancylostoma* spp.; *Uncinaria stenocephala* of *Uncinaria* spp.; and *Bunostomum phlebotomum* and *Bunostomum trigonocephalum* of *Bunostomum* spp.

(5) Nematodes of the order *Strongylida*

(a) nematodes of the family *Angiostrongylidae,* for example, *Aelurostrongylus abstrusus* of *Aelurostrongylus* spp.; and *Angiostrongylus vasorum* and *Angiostrongylus cantonesis* of *Angiostrongylus* spp.;

(b) nematodes of the family *Crenosomatidae,* for example, *Crenosoma aerophila* and *Crenosoma vulpis* of *Crenosoma* spp.;

(c) nematodes of the family *Filaroididae,* for example, *Filaroides hirthi* and *Filaroides osleri* of *Filaroides* spp.;

(d) lungworms of the family *Metastrongylidae,* for example, *Metastrongylus apri, Metastrongylus asymmetricus, Metastrongylus pudendotectus,* and *Metastrongylus salmi* of *Metastrongylus* spp.;

(e) gapeworms of the family *Syngamidae,* for example, *Cyathostoma bronchialis* of *Cyathostoma* spp.; and *Syngamus skrjabinomorpha* and *Syngamus trachea* of *Syngamus* spp.

(6) Nematodes of the order *Strongylida*

(a) nematodes of the family *Molineidae,* for example, *Nematodirus filicollis* and *Nematodirus spathiger* of *Nematodirus* spp.;
(b) nematodes of the family *Dictyocaulidae,* for example, *Dictyocaulus filaria* and *Dictyocaulus viviparus* of *Dictyocaulus* spp.;
(c) nematodes of the family *Haemonchidae,* for example, *Haemonchus contortus* of *Haemonchus* spp.; and *Mecistocirrus digitatus* of *Mecistocirrus* spp.;
(d) nematodes of the family *Haemonchidae,* for example, *Ostertagia ostertagi* of *Ostertagia* spp.;
(e) nematodes of the family *Heligmonellidae,* for example, *Nippostrongylus braziliensis* of *Nippostrongylus* spp.;
(f) nematodes of the family *Trichostrongylidae,* for example, *Trichostrongylus axel, Trichostrongylus colubriformis,* and *Trichostrongylus tenuis* of *Trichostrongylus* spp.; *Hyostrongylus rubidus* of *Hyostrongylus* spp.; and *Obeliscoides cuniculi* of *Obeliscoides* spp.

(7) Nematodes of the order *Strongylida*

(a) nematodes of the family *Chabertiidae,* for example, *Chabertia ovina* of *Chabertia* spp.; and *Oesophagostomum brevicaudatum, Oesophagostomum columbianum, Oesophagostomum dentatum, Oesophagostomum georgianum, Oesophagostomum maplestonei, Oesophagostomum quadrispinulatum, Oesophagostomum radiatum, Oesophagostomum venulosum,* and *Oesophagostomum watanabei* of *Oesophagostomum* spp.;
(b) nematodes of the family *Stephanuridae,* for example, *Stephanurus dentatus* of *Stephanurus* spp.;
(c) nematodes of the family *Strongylidae,* for example, *Strongylus asini, Strongylus edentatus, Strongylus equinus,* and *Strongylus vulgaris* of *Strongylus* spp.

(8) Nematodes of the order *Oxyurida*
nematodes of the family *Oxyuridae,* for example, *Enterobius anthropopitheci* and *Enterobius vermicularis* of *Enterobius* spp.; *Oxyuris equi* of *Oxyuris* spp.; and *Passalurus ambiguus* of *Passalurus* spp.
(9) Nematodes of the order *Ascaridida*

(a) nematodes of the family *Ascaridiidae,* for example, *Ascaridia galli* of *Ascaridia* spp.;
(b) nematodes of the family *Heterakidae,* for example, *Heterakis beramporia, Heterakis brevispiculum, Heterakis gallinarum, Heterakis pusilla,* and *Heterakis putaustralis* of *Heterakis* spp.;
(c) nematodes of the family *Anisakidae,* for example, *Anisakis simplex* of *Anisakis* spp.;
(d) nematodes of the family Ascarididae, for example, *Ascaris lumbricoides* and *Ascaris suum* of *Ascaris* spp.; and *Parascaris equorum* of *Parascaris* spp.;
(e) nematodes of the family *Toxocaridae,* for example, *Toxocara canis, Toxocara leonina, Toxocara suum, Toxocara vitulorum,* and *Toxocara cati* of *Toxocara* spp.

(10) Nematodes of the order *Spirurida*

(a) nematodes of the family *Onchocercidae,* for example, *Brugia malayi, Brugia pahangi,* and *Brugia patei* of *Brugia* spp.; *Dipetalonema recondition* of *Dipetalonema* spp.; *Dirofilaria immitis* of *Dirofilaria* spp.; *Filaria oculi* of *Filaria* spp.; and *Onchocerca cervicalis, Onchocerca gibsoni,* and *Onchocerca gutturosa* of *Onchocerca* spp.;
(b) nematodes of the family *Setariidae,* for example, *Setaria digitata, Setaria equina, Setaria labiatopapillosa,* and *Setaria marshalli* of *Setaria* spp.; and *Wuchereria bancrofti* of *Wuchereria* spp.;
(c) nematodes of the family *Filariidae,* for example, *Parafilaria multipapillosa* of *Parafilaria* spp.; and *Stephanofilaria assamensis, Stephanofilaria dedoesi, Stephanofilaria kaeli, Stephanofilaria okinawaensis,* and *Stephanofilaria stilesi* of *Stephanofilaria* spp.

(11) Nematodes of the order *Spirurida*

(a) nematodes of the family *Gnathostomatidae,* for example, *Gnathostoma doloresi* and *Gnathostoma spinigerum* of *Gnathostoma* spp.;
(b) nematodes of the family *Habronematidae,* for example, *Habronema majus, Habronema microstoma,* and *Habronema muscae* of *Habronema* spp.; and *Draschia megastoma* of *Draschia* spp.;
(c) nematodes of the family *Physalopteridae,* for example, *Physaloptera canis, Physaloptera cesticillata, Physaloptera erdocyona, Physaloptera felidis, Physaloptera gemina, Physaloptera papilloradiata, Physaloptera praeputialis, Physaloptera pseudopraerutialis, Physaloptera rara, Physaloptera sibirica,* and *Physaloptera vulpineus* of *Physaloptera* spp.;
(d) nematodes of the family *Gongylonematidae,* for example, *Gongylonema pulchrum* of *Gongylonema* spp.;

(e) nematodes of the family *Spirocercidae,* for example, *Ascarops strongylina* of *Ascarops* spp.;
(f) nematodes of the family *Thelaziidae,* for example, *Thelazia callipaeda, Thelazia gulosa, Thelazia lacrymalis, Thelazia rhodesi,* and *Thelazia skrjabini* of *Thelazia* spp.

[Control agent for other pests]

The imidazo[1,2-a]pyridine compound of the present invention is additionally excellent in control effect on insect pests that have a stinger or venom and harm humans and animals, insect pests that mediate various pathogens or disease-causing microbes, or insect pests that cause discomfort to humans (toxic pests, hygienic pests, and obnoxious pests, etc.).
Specific examples thereof will be shown below.

(1) Insect pests of the order *Hymenoptera*
bees of the family *Argidae,* bees of the family *Cynipidae,* bees of the family *Diprionidae,* ants of the family *Formicidae,* bees of the family *Mutillidae,* and bees of the family *Vespidae.*
(2) Other insect pests
*Blattodea,* termites, *Araneae,* centipedes, millipedes, crustacea, and *Cimex lectularius.*

[Pharmaceutical formulation]

Some pharmaceutical formulations of the pest control agent, the insecticide or acaracide, the ectoparasite control agent or the endoparasite control agent or expellant of the present invention will be shown. However, additives and addition ratios should not be limited by these examples and may be changed in wide ranges. The term "part" in the pharmaceutical formulations represents part by weight.
Hereinafter, the pharmaceutical formulations for agriculture or horticulture and for paddy rice will be shown.

(Preparation 1: Wettable powder)

40 parts of the imidazo[1,2-a]pyridine compound of the present invention, 53 parts of diatomaceous earth, 4 parts of higher alcohol sulfuric acid ester, and 3 parts of alkyl naphthalenesulfonate are uniformly mixed and finely milled to obtain a wettable powder containing 40% of the active ingredient.

(Preparation 2: Emulsion)

30 parts of the imidazo[1,2-a]pyridine compound of the present invention, 33 parts of xylene, 30 parts of dimethylformamide, and 7 parts of polyoxyethylene alkyl allyl ether are mixed and dissolved to obtain an emulsion containing 30% of the active ingredient.

(Preparation 3: Granular formulation)

5 parts of the imidazo[1,2-a]pyridine compound of the present invention, 40 parts of talc, 38 parts of clay, 10 parts of bentonite, and 7 parts of sodium alkyl sulfate are uniformly mixed, finely milled, and then granulated into a granular shape of 0.5 to 1.0 mm in diameter to obtain a granular formulation containing 5% of the active ingredient.

(Preparation 4: Granular formulation)

5 parts of the imidazo[1,2-a]pyridine compound of the present invention, 73 parts of clay, 20 parts of bentonite, 1 part of dioctyl sulfosuccinate sodium salt, and 1 part of potassium phosphate are well milled and mixed, and after addition of water, the mixture is well kneaded, then granulated, and dried to obtain a granular formulation containing 5% of the active ingredient.

(Preparation 5: Suspension)

10 parts of the imidazo[1,2-a]pyridine compound of the present invention, 4 parts of polyoxyethylene alkyl allyl ether, 2 parts of polycarboxylic acid sodium salt, 10 parts of glycerin, 0.2 parts of xanthan gum, and 73.8 parts of water are mixed and wet-milled until the particle size becomes 3 microns or smaller to obtain a suspension containing 10% of the active ingredient.
Hereinafter, the pharmaceutical formulations of the ectoparasite control agent or the endoparasite control agent

or expellant will be shown.

(Preparation 6: Granules)

5 parts of the imidazo[1,2-a]pyridine compound of the present invention are dissolved in an organic solvent to obtain a solution. The solution is sprayed onto 94 parts of kaolin and 1 part of white carbon. Then, the solvent is evaporated under reduced pressure. This type of granules may be mixed with animal feed.

(Preparation 7: Injectable filler)

0.1 to 1 parts of the imidazo[1,2-a]pyridine compound of the present invention and 99 to 99.9 parts of peanut oil are uniformly mixed and then sterilized by filtration through a sterilizing filter.

(Preparation 8: Pore-on formulation)

5 parts of the imidazo[1,2-a]pyridine compound of the present invention, 10 parts of myristic acid ester, and 85 parts of isopropanol are uniformly mixed to obtain a pore-on formulation.

(Preparation 9: Spot-on formulation)

10 to 15 parts of the imidazo[1,2-a]pyridine compound of the present invention, 10 parts of palmitic acid ester, and 75 to 80 parts of isopropanol are uniformly mixed to obtain a spot-on formulation.

(Preparation 10: Spray formulation)

1 part of the imidazo[1,2-a]pyridine compound of the present invention, 10 parts of propylene glycol, and 89 parts of isopropanol are uniformly mixed to obtain a spray formulation.

Next, the present invention will be more specifically described with reference to Examples. However, the present invention is not limited by the following Examples by any means.

[Example 1]

Synthesis of 3-(ethylsulfonyl)-2-(1-methyl-5-(4-(trifluoromethoxy)phenyl)-1H-imidazol-2-yl)-6-(trifluoromethyl)imidazo[1,2-a]pyridine (compound No. a-3)

(Step 1) Synthesis of 2-bromo-3-chloro-6-(trifluoromethyl)imidazo[1,2-a]pyridine

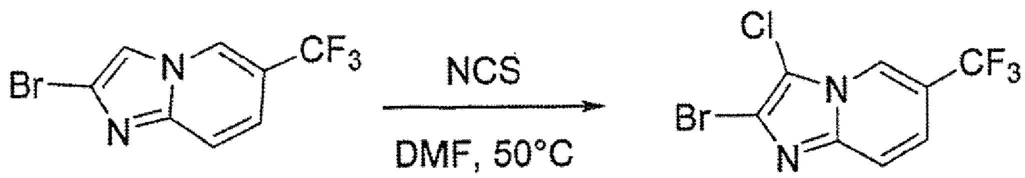


2-Bromo-6-(trifluoromethyl)imidazo[1,2-a]pyridine (1.98 g) was dissolved in N,N-dimethylformamide (15 ml). N- chlorosuccinimide (1.1 g) was added thereto, and the mixture was stirred at 50°C for 1 hour. The obtained solution was poured to water, followed by extraction with ethyl acetate. The obtained organic layer was washed with saturated saline, dried over anhydrous magnesium sulfate, and filtered. The filtrate was concentrated under reduced pressure, and the obtained residue was purified by silica gel column chromatography to obtain 1.6 g of the title compound (yield: 71%).

$^1$H-NMR of the obtained title compound will be shown below.

$^1$H-NMR (400 MHz, $CDCl_3$) δ: 8.41 (1H, s), 7.69 (1H, d), 7.43 (1H, dd).

(Step 2) Synthesis of 3-Chloro-2-(1-methyl-1H-imidazol-2-yl)-6-(trifluoromethyl)imidazo[1,2-a]pyridine

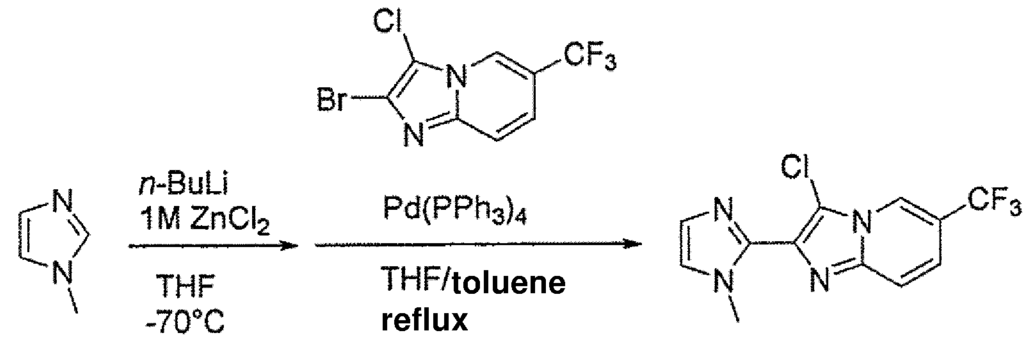


1-Methyl-1H-imidazole (0.33 g) was dissolved in tetrahydrofuran (14 ml), and the reaction vessel was purged with nitrogen, followed by cooling to -70°C. A solution of 2.75 M n-butyllithium in n-hexane solution (1.5 ml) was added dropwise thereto, and the mixture was stirred at - 70°C for 30 minutes. A 1 M zinc chloride (II) tetrahydrofuran solution (8 ml) was added thereto, and the mixture was heated to room temperature, and stirred for 1 hour. Thereafter, a solution of 2-bromo-3-chloro-6-(trifluoromethyl)imidazo[1,2-a]pyridine (1.2 g) in toluene (27 ml) and tetrakis(triphenylphosphine)palladium (0) (0.16 g) were added, and the reaction vessel was purged with nitrogen, followed by stirring overnight under heating to reflux. The obtained solution was poured to water, followed by extraction with ethyl acetate. The obtained organic layer was washed with saturated saline, dried over anhydrous magnesium sulfate, and filtered. The filtrate was concentrated under reduced pressure, and the obtained residue was purified by silica gel column chromatography to obtain 0.70 g of the title compound (yield: 58%).

$^1$H-NMR of the obtained title compound will be shown below.

$^1$H-NMR (400 MHz, $CDCl_3$) δ: 8.56-8.51 (1H, m), 7.72 (1H, d), 7.43 (1H, dd), 7.27-7.25 (1H, m), 7.02 (1H, d), 4.13 (3H, s) .

(Step 3) Synthesis of 3-(ethylthio)-2-(1-methyl-1H-imidazol-2-yl)-6-(trifluoromethyl)imidazo[1,2-a]pyridine

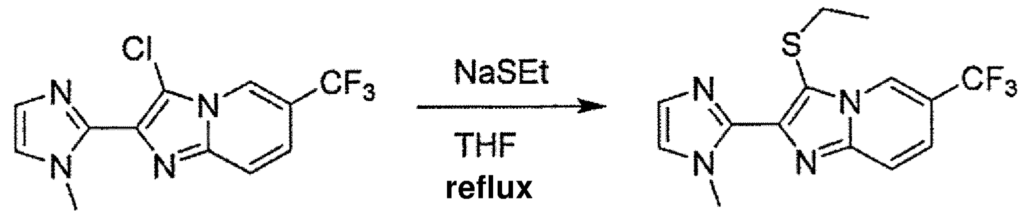


3-Chloro-2-(1-methyl-1H-imidazol-2-yl)-6-(trifluoromethyl)imidazo[1,2-a]pyridine (0.70 g) was dissolved in tetrahydrofuran (10 ml), and mixture was stirred at room temperature. 80% Ethylmercaptan sodium (1.2 g) was added thereto, and the mixture was stirred for 4 hours under heating to reflux. The obtained solution was poured to water, followed by extraction with ethyl acetate. The obtained organic layer was washed with saturated saline, dried over anhydrous magnesium sulfate, and filtered. The filtrate was concentrated under reduced pressure, and the obtained residue was purified by silica gel column chromatography to obtain 0.29 g of the title compound (yield: 38%).

$^1$H-NMR of the obtained title compound will be shown below.

$^1$H-NMR (400 MHz, $CDCl_3$) δ: 8.95 (1H, s), 7.72 (1H, d), 7.44 (1H, dd), 7.25 (1H, d), 7.03 (1H, d), 4.05 (3H, s), 3.01 (2H, q), 1.16 (3H, t).

(Step 4) Synthesis of 2-(5-bromo-1-methyl-1H-imidazol-2-yl)-3-(ethylthio)-6-(trifluoromethyl)imidazo[1,2-a]pyridine

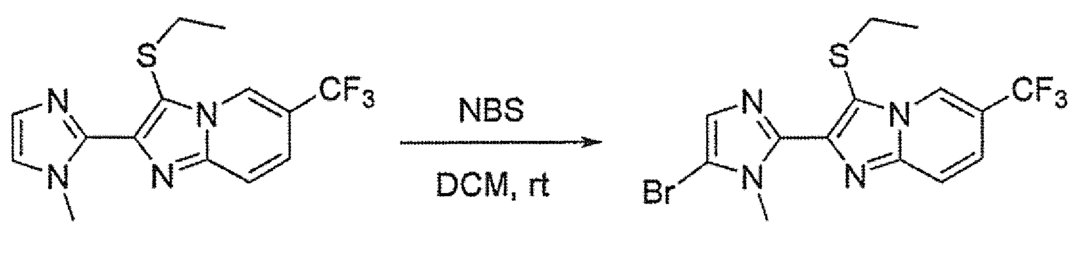

3-(Ethylthio)-2-(1-methyl-1H-imidazol-2-yl)-6-(trifluoromethyl)imidazo[1,2-a]pyridine (0.29 g) was dissolved in dichloromethane (10 ml), and the mixture was cooled to 0°C. N-bromosuccinimide (0.87 g) was added thereto, and the mixture was stirred at room temperature for 3 hours. The obtained solution was poured to water, followed by extraction with dichloromethane. The obtained organic layer was washed with a saturated solution of sodium bicarbonate, dried over anhydrous magnesium sulfate, and filtered. The filtrate was concentrated under reduced pressure, and the obtained residue was used for the next step without being purified.

(Step 5) Synthesis of 2-(5-bromo-1-methyl-1H-imidazol-2-yl)-3-(ethylsulfonyl)-6-(trifluoromethyl)imidazo[1,2-a]pyridine

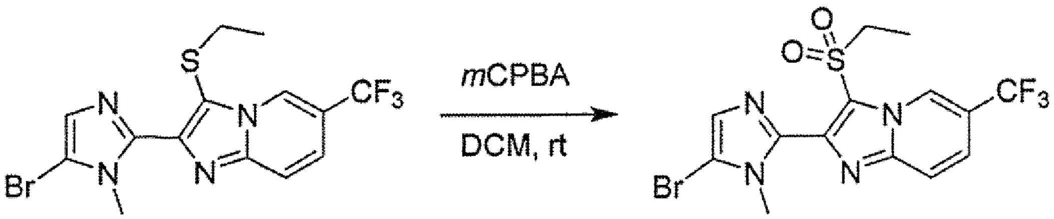


The 2-(5-bromo-1-methyl-1H-imidazol-2-yl)-3-(ethylthio)-6-(trifluoromethyl)imidazo[1,2-a]pyridine obtained in step 4 was dissolved in dichloromethane (10 ml), and the mixture was cooled to 0°C, and stirred. 70% m-Chloroperbenzoic acid (0.48 g) was added thereto, and the mixture was stirred overnight at room temperature. The obtained solution was poured to a mixed solution of a saturated aqueous solution of sodium bicarbonate and a saturated aqueous solution of sodium thiosulfate, followed by extraction with dichloromethane. The obtained organic layer was washed with saturated saline, dried over anhydrous magnesium sulfate, and filtered. The filtrate was concentrated under reduced pressure, and the obtained residue was purified by silica gel column chromatography to obtain 0.35 g of the title compound (yield: 90%, two steps).

$^1$H-NMR of the obtained title compound will be shown below.

$^1$H-NMR (400 MHz, $CDCl_3$) $\delta$: 9.62 (1H, s), 7.85 (1H, d), 7.67-7.62 (1H, m), 7.20 (1H, s), 4.02 (2H, q), 3.89 (3H, s), 1.40 (3H, t) .

(Step 6) Synthesis of 3-(ethylsulfonyl)-2-(1-methyl-5-(4-(trifluoromethoxy)phenyl)-1H-imidazol-2-yl)-6-(trifluoromethyl)imidazo[1,2-a]pyridine

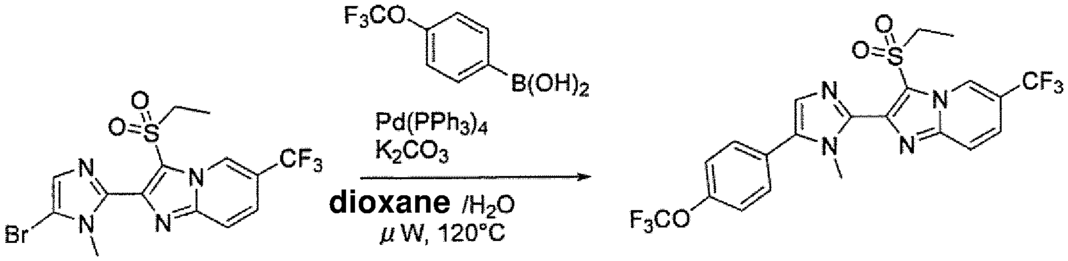


2-(5-Bromo-1-methyl-1H-imidazol-2-yl)-3-(ethylsulfonyl)-6-(trifluoromethyl)imidazo[1,2-a]pyridine (0.10 g) and dioxane (4 ml) were added to a microwave synthesis reaction vessel. 4-Trifluoromethoxyphenylboronic acid (0.061 g), tetrakis(triphenylphosphine)palladium (0) (0.013 g), potassium carbonate (0.048 g) and water (0.5 ml) were added thereto, and the mixture was reacted at 120°C for 1 hour using a microwave synthesis apparatus. The obtained solution was poured to water, followed by extraction with ethyl acetate. The obtained organic layer was washed with saturated saline, dried over anhydrous magnesium sulfate, and filtered. The filtrate was concentrated under reduced pressure, and the obtained residue was purified by silica gel column chromatography to obtain 0.050 g of the title compound (yield: 42%).

$^1$H-NMR of the obtained title compound will be shown below.

$^1$H-NMR (400 MHz, $CDCl_3$) $\delta$: 9.65 (1H, s), 7.87 (1H, d), 7.65 (1H, dd), 7.53-7.47 (2H, m), 7.34 (2H, d), 7.28-7.25 (1H, m), 4.11 (2H, q), 3.86 (3H, s), 1.43 (3H, t).

Examples of the imidazo[1,2-a]pyridine compounds of the present invention produced in the same way as in

Examples described above are shown in Table 1. The physical property data of the compounds is described in the columns of "physical properties". Properties or melting points (m.p.) are described as physical property data.

[Table 1]

| Compound No. | Structure | Physical properties |
|---|---|---|
| a-1 | 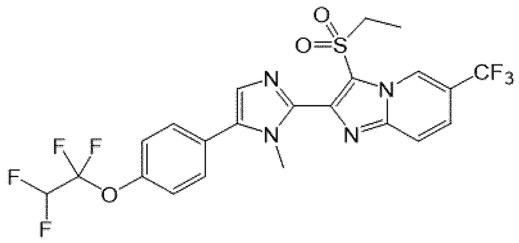 | m.p.226-228(°C) |
| a-2 | 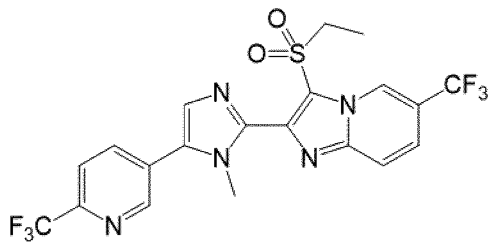 | m.p.224-226(°C) |
| a-3 | 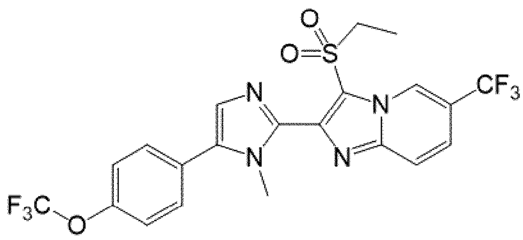 | m.p.158-160(°C) |
| a-5 | 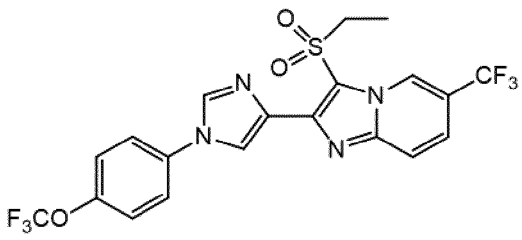 | m.p.215-217(°C) |
| a-6 | 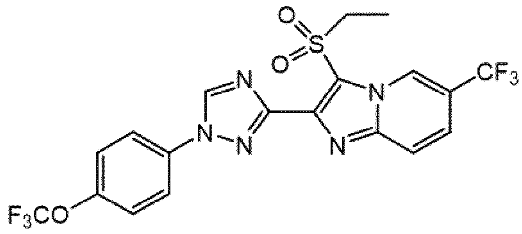 | m.p.256-258(°C) |
| a-7 | 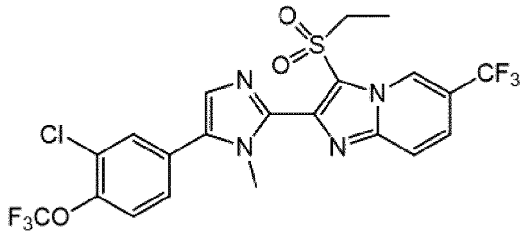 | m.p.195-197(°C) |

(continued)

| Compound No. | Structure | Physical properties |
|---|---|---|
| a-8 | 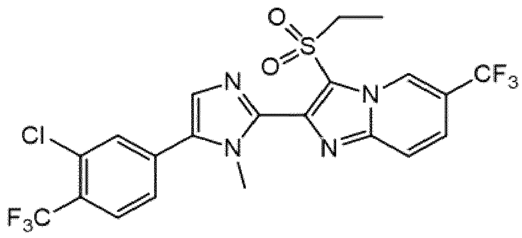 | m.p.216-218(°C) |
| a-9 | 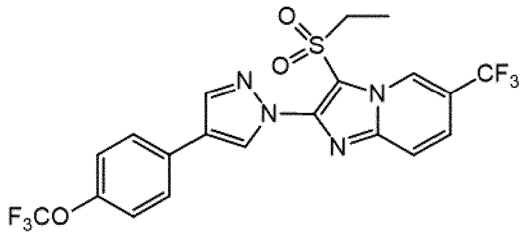 | m.p.196-198(°C) |
| a-10 | 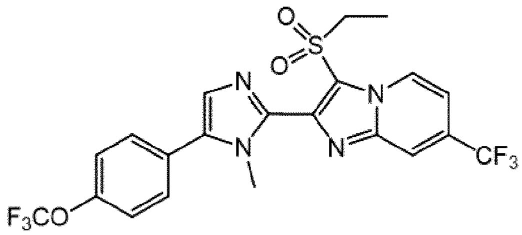 | m.p.203-205(°C) |
| a-11 | 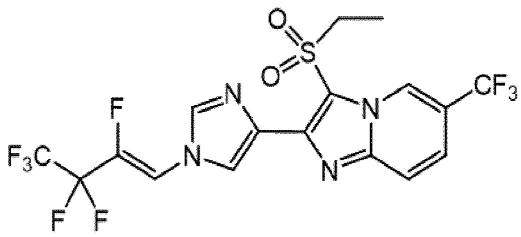 | m.p.244-246(°C) |
| a-12 | 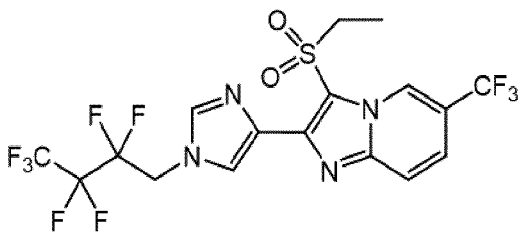 | m.p.176-178(°C) |

[Table 2]

| Compound No. | Structure | Physical properties |
|---|---|---|
| a-4 | 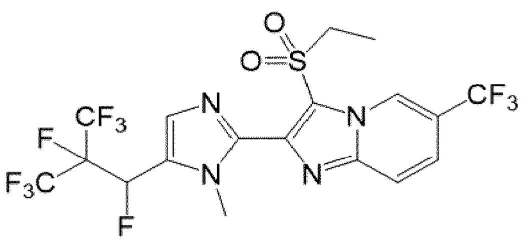 | m.p.162-164(°C) |

(continued)

| Compound No. | Structure | Physical properties |
|---|---|---|
| a-13 | 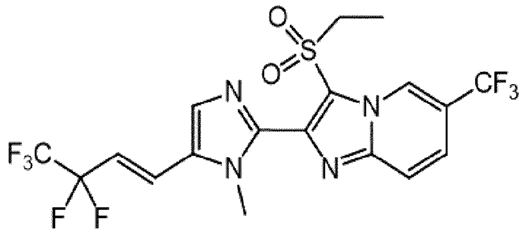 | m.p.194-196(°C) |
| a-14 | 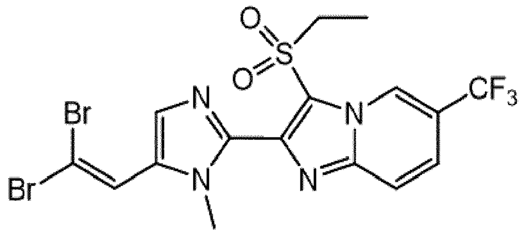 | m.p.233-235(°C) |
| a-15 | 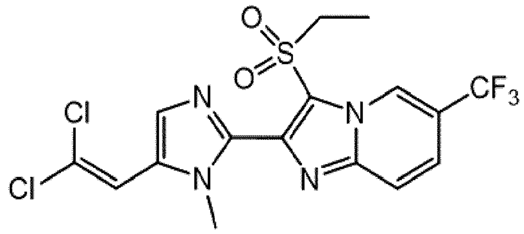 | m.p.236-238(°C) |
| a-16 | 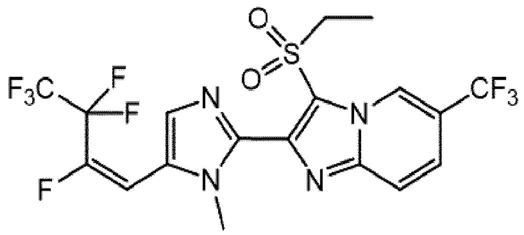 | m.p.169-171(°C) |
| a-17 | 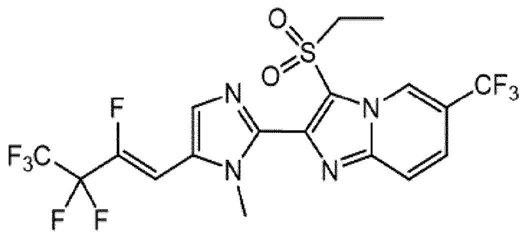 | m.p.182-184(°C) |
| a-18 | 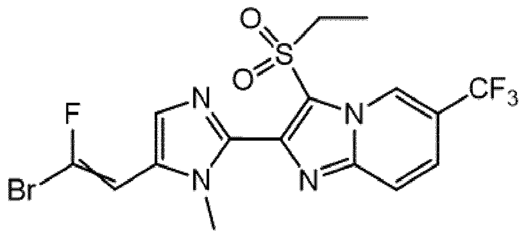 | m.p.216-218(°C) |

(continued)

| Compound No. | Structure | Physical properties |
|---|---|---|
| a-19 | 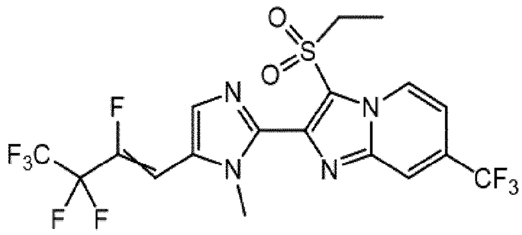 | m.p.156-158(°C) |
| a-20 | 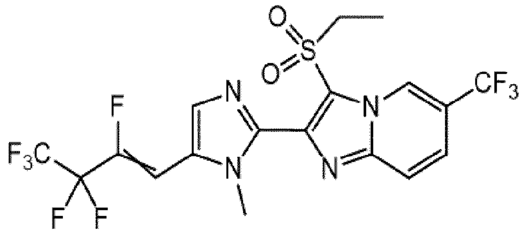 | m.p.173-175(°C) |
| a-21 | 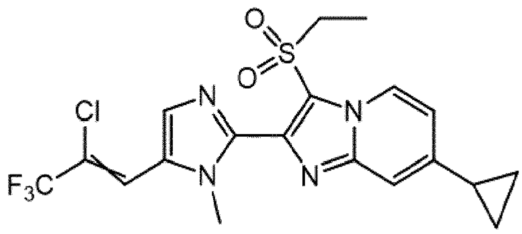 | white solid |
| a-22 | 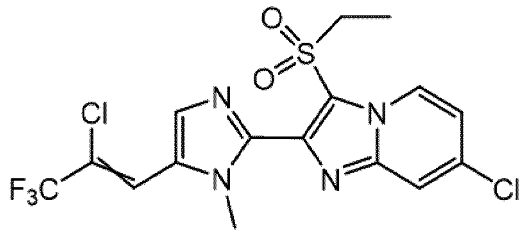 | m.p.205-210(°C) |
| a-23 | 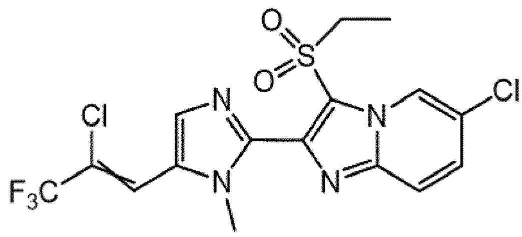 | m.p.276-281(°C) |
| a-24 | 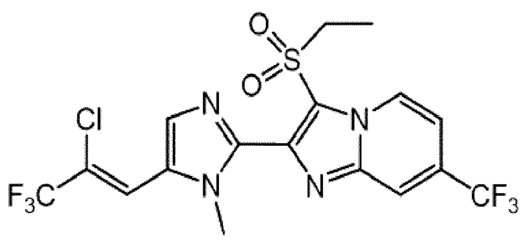 | white solid |

[Biological test]

Test Examples given below show that the imidazo[1,2-a]pyridine compound of the present invention is useful as an active ingredient for pest control agents and ectoparasite control agents. The term "part" is based on weight.

(Preparation of test emulsion)

5 parts of the imidazo[1,2-a]pyridine compound of the present invention, 93.6 parts of dimethylformamide, and 1.4 parts of polyoxyethylene alkyl aryl ether were mixed and dissolved to prepare emulsion (I) containing 5% of the active ingredient.
For a control, 98.5 parts of dimethylformamide and 1.5 parts of polyoxyethylene alkyl aryl ether were mixed and dissolved to prepare emulsion (II).
An insecticidal rate was calculated according to the following expression:

$$\text{Insecticidal rate (\%)} = (\text{The number of dead insects} / \text{The number of tested insects}) \times 100$$

(Test Example 1) Test of efficacy on *Plutella xylostella*

The emulsion (I) was diluted with water such that the concentration of the compound of the present invention was 125 ppm. Cabbage leaves were dipped in the dilution for 30 seconds. The resulting cabbage leaves were placed in a petri dish. Five second instar larvae of *Plutella xylostella* were released thereto. The petri dish was placed in a thermostat chamber having a temperature of 25°C and a humidity of 60%. Life and death were determined after 3 days from the release of the insects, and the insecticidal rate was calculated. The test was conducted in duplicate.
Compounds of compound Nos. a-1 to a-13, a-17 and a-19 to a-24 were tested for their efficacy on *Plutella xylostella.* All the compounds exhibited an insecticidal rate of 80% or more for *Plutella xylostella.*

(Test Example 2) Test of efficacy on *Phyllotreta striolata*

The emulsion (I) was diluted with water such that the concentration of the compound of the present invention was 125 ppm to prepare a test chemical. The test chemical was sprayed to Qing geng cai seedlings (at the seventh true leaf stage) planted in 10-cm pots. The Qing geng cai seedlings were dried in air and then placed in a plastic cup. Ten *Phyllotreta striolata* adults were released thereto. The plastic cup was stored in a thermostat chamber having a temperature of 25°C and a humidity of 65%. Life and death were determined after 7 days from the release of the insects, and the insecticidal rate was calculated. The test was conducted in duplicate.
Compounds of compound Nos. a-2, a-3, a-5 to a-7, a-10 to a-13, a-16, a-17, and a-19 to a-23 were tested for their efficacy on *Phyllotreta striolata* adults. All the compounds exhibited an insecticidal rate of 80% or more for *Phyllotreta striolata* adults.

(Test Example 3) Test of efficacy on *Aphis gossypii*

Cucumber seeds were planted in 10-cm pots. *Aphis gossypii* female adults were released to the cucumbers 10 days after their germination. On the following day, the female adults were removed while the born first instar larvae were saved.
The emulsion (I) was diluted with water such that the concentration of the compound of the present invention was 125 ppm, and the dilution was sprayed to the cucumbers.
Thereafter, the cucumbers were stored in a thermostat chamber having a temperature of 25°C and a humidity of 60% during the test period, life and death were determined after 5 days, and the insecticidal rate was calculated. The test was conducted in duplicate.
Compounds of compound Nos. a-3, a-5, a-7, a-11, a-13, a-16, a-17 and a-20 were tested for their efficacy on *Aphis gossypii* adults. All the compounds exhibited an insecticidal rate of 80% or more for *Aphis gossypii* adults.

(Test Example 4) Test of efficacy on cat flea

The compound of the present invention was dissolved in isopropanol to prepare a chemical at 20 ppm. 100 μL of the chemical was applied to the bottom surface of the inside of a glass vial (φ330 mm), and the isopropanol was volatilized by air drying to form a thin film of the benzoimidazole compound of the present invention on the bottom surface.
Five adults (mixed males and females) of cat flea (*Ctenocephalides felis*) were released to a vial. The vial was lidded, and placed in a thermostat chamber at 25°C. Life and death of the cat flea were determined after 4 days from the release of the insects, and the insecticidal rate was calculated. The test was conducted in duplicate.
Compounds of compound Nos. a-3, a-5, a-9, a-13 and a-17 to a-20 were tested for their efficacy on cat flea

adults. All the compounds exhibited an insecticidal rate of 80% or more for cat flea adults.

All the compounds selected at random from among the imidazo[1,2-a]pyridine compounds of the present invention exerted the effect as described above. It may therefore be understood that the imidazo[1,2-a]pyridine compound of the present invention, including unillustrated compounds, is a compound having an effect such as a pest control effect, particularly, a acaracidal or insecticidal effect. It may also be understood that the imidazo[1,2-a]pyridine compound of the present invention is a compound also having an effect on parasites harmful to humans and animals, such as ectoparasites.

## Claims

1. A compound of any of formulae (I) to (III) or a salt thereof:

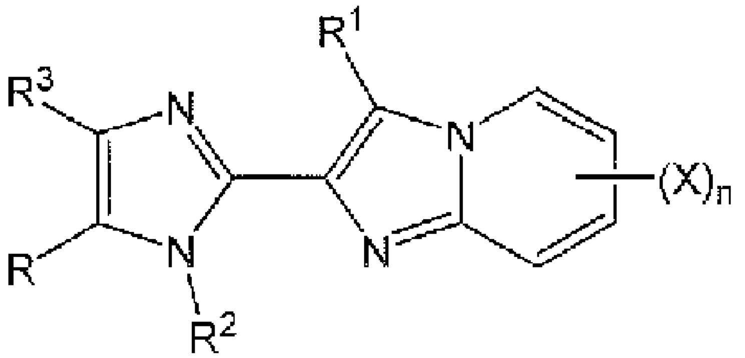

(I)

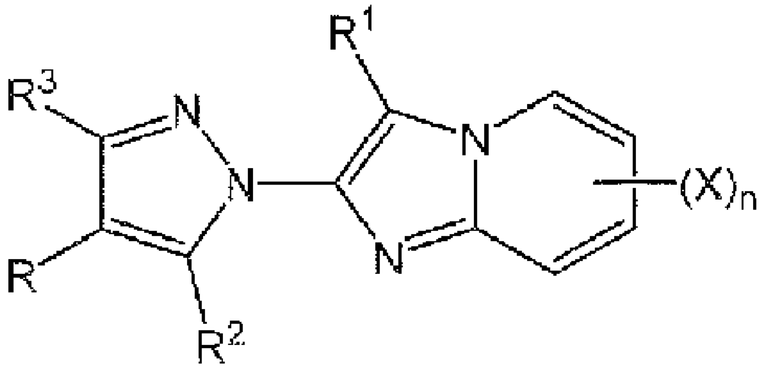

(II)

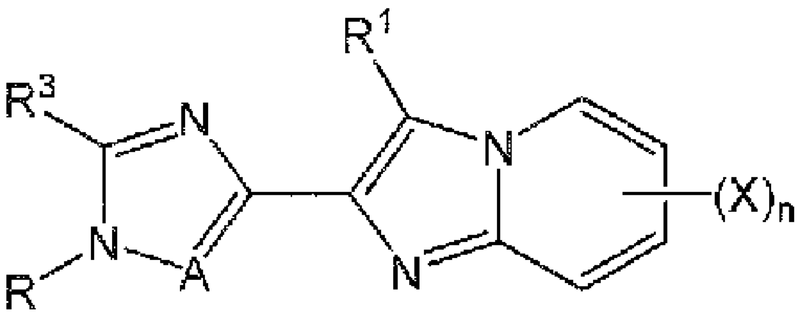

(III)

wherein

$R^1$ represents a substituted or unsubstituted C1-6 alkylsulfonyl group;
$R^2$ and $R^3$ each independently represent a hydrogen atom, a substituted or unsubstituted C1-6 alkyl group or a halogeno group;
R represents a substituted or unsubstituted C1-6 alkyl group, a substituted or unsubstituted C2-6 alkenyl group, a substituted or unsubstituted C6-10 aryl group or a substituted or unsubstituted 5- to 6-membered heteroaryl

group;
X represents a substituted or unsubstituted C1-6 alkyl group, a substituted or unsubstituted C2-6 alkenyl group, a substituted or unsubstituted C2-6 alkynyl group, a hydroxyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C6-10 aryl group, a substituted or unsubstituted 5- to 6-membered heteroaryl group, a substituted or unsubstituted C6-10 aryloxy group, a substituted or unsubstituted 5- to 6-membered heteroaryloxy group, a substituted or unsubstituted amino group, a substituted or unsubstituted aminocarbonyl group, a substituted or unsubstituted hydrazinyl group, a nitro group, a cyano group or a halogeno group;
n represents a number of chemically acceptable Xs, and is an integer of 0 to 4; Xs are the same or different when n is 2 or more; and
A represents a nitrogen atom or $CR^2$.

**2.** A pest control agent comprising at least one active ingredient selected from the group consisting of a compound according to claim 1 and a salt thereof.

**3.** An insecticide or acaracide comprising at least one active ingredient selected from the group consisting of a compound according to claim 1 and a salt thereof.

**4.** An ectoparasite control agent comprising at least one active ingredient selected from the group consisting of a compound according to claim 1 and a salt thereof.

**5.** An endoparasite control agent or expellant comprising at least one active ingredient selected from the group consisting of a compound according to claim 1 and a salt thereof.

**INTERNATIONAL SEARCH REPORT**

International application No. **PCT/JP2021/034331**

**A. CLASSIFICATION OF SUBJECT MATTER**

***C07D 471/04***(2006.01)i; ***A01M 1/20***(2006.01)i; ***A01N 43/90***(2006.01)i; ***A01N 47/02***(2006.01)i; ***A01P 7/04***(2006.01)i; ***A61K 31/437***(2006.01)i; ***A61K 31/444***(2006.01)i; ***A61P 33/00***(2006.01)i; ***A61P 33/10***(2006.01)i; ***A61P 33/14***(2006.01)i
FI: C07D471/04 108Q; A01M1/20 A; A01N43/90 103; A01N47/02; A01P7/04; A61K31/437; A61K31/444; A61P33/00; A61P33/10; A61P33/14; C07D471/04 CSP

According to International Patent Classification (IPC) or to both national classification and IPC

**B. FIELDS SEARCHED**

Minimum documentation searched (classification system followed by classification symbols)

C07D471/04; A01M1/20; A01N43/90; A01N47/02; A01P7/04; A61K31/437; A61K31/444; A61P33/00; A61P33/10; A61P33/14

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2017/104741 A1 (NIPPON SODA CO., LTD) 22 June 2017 (2017-06-22) paragraph [0261], table 18, compound no. 15-17, paragraphs [0295]-[0297], test example 9, paragraphs [0307]-[0309], test example 14, paragraphs [0105]-[0106], [0321]-[0322] | 1-5 |
| A | JP 2020-079324 A (SUMITOMO CHEMICAL CO) 28 May 2020 (2020-05-28) claims, paragraphs [0265]-[0329] | 1-5 |
| A | JP 2018-115180 A (SUMITOMO CHEMICAL CO) 26 July 2018 (2018-07-26) claims, paragraphs [0317]-[0595] | 1-5 |
| A | JP 2014-509616 A (EMBLEM TECHNOLOGY TRANSFER GMBH) 21 April 2014 (2014-04-21) claims, paragraphs [0232]-[0272] | 1-5 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 November 2021** | **22 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/034331**

| Patent document cited in search report | Publication date (day/month/year) | Patent family member(s) | Publication date (day/month/year) |
|---|---|---|---|
| WO 2017/104741 A1 | 22 June 2017 | US 2018/0362470 A1<br>paragraph [0653], no. 15-7, paragraphs [0678]-[0679], test example 9, paragraphs [0688]-[0689], experimental example 14, paragraphs [0220]-[0223], [0700]-[0701]<br>EP 3395801 A1<br>CN 108430976 A<br>KR 10-2018-0095813 A | |
| JP 2020-079324 A | 28 May 2020 | (Family: none) | |
| JP 2018-115180 A | 26 July 2018 | (Family: none) | |
| JP 2014-509616 A | 21 April 2014 | WO 2012/130322 A1<br>claims, pp. 87-110<br>US 2014/0221354 A1<br>EP 2691392 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017104741 A


### Non-patent literature cited in the description


- **COLBY. S.R.** Calculating Synergistic and Antagonistic Responses of Herbicide Combinations. *Weeds,* 1967, vol. 15, 20-22
- *CHEMICAL ABSTRACTS,* 943137-49-3